(19) Europäisches Patentamt
European Patent Office
Office européen des brevets

(11) Veröffentlichungsnummer: **0 062 236 A2**

(12) **EUROPÄISCHE PATENTANMELDUNG**

(21) Anmeldenummer: **82102446.0**

(22) Anmeldetag: **24.03.82**

(51) Int. Cl.³: **C 07 D 405/06**, A 01 N 43/64 // C07D319/06

(30) Priorität: **04.04.81 DE 3113628**

(43) Veröffentlichungstag der Anmeldung: **13.10.82 Patentblatt 82/41**

(84) Benannte Vertragsstaaten: **AT BE CH DE FR GB IT LI LU NL SE**

(71) Anmelder: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Feuerherd, Karl-Heinz, Dr., c/o BASF Japan LTD. C.P.O. Box 1757, Tokyo 100-91 (JP)**
Erfinder: **Meyer, Norbert, Dr., Stahlbuehlring 155A, D-6802 Ladenburg (DE)**
Erfinder: **Goetz, Norbert, Dr., Schoefferstrasse 25, D-6250 Worms (DE)**
Erfinder: **Pommer, Ernst-Heinrich, Dr., Berliner Platz 7, D-6703 Limburgerhof (DE)**

(54) **Fungizide Mittel, enthaltend Triazolylderivate und Verfahren zur Bekämpfung von Pilzen mit ihnen.**

(57) Die vorliegende Erfindung betrifft fungizide Mittel, welche Triazolylderivate der Formel

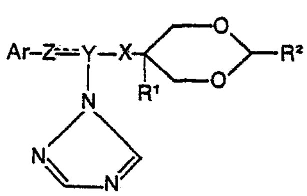

I

worin Ar, Z, Y, X, $R^1$ und $R^2$ die in der Beschreibung angegebene Bedeutung besitzt, enthalten.

EP 0 062 236 A2

# Fungizide Mittel, enthaltend Triazolylderivate und Verfahren zur Bekämpfung von Pilzen mit ihnen

Die vorliegende Erfindung betrifft fungizide Mittel, die Triazolylderivate oder deren Salze oder Metallkomplexverbindungen enthalten sowie Verfahren zur Bekämpfung von phytopathogenen Pilzen mit ihnen.

Es ist bekannt, daß Triazolderivate wie beispielsweise 2,2-Dimethyl-4-(1,2,4-triazol-1-yl)-5-phenylpentan-3-on (vgl. DE-OS 26 38 470) fungizide Wirksamkeit besitzen. Die Wirkung dieser Stoffe ist bei niedrigen Aufwandmengen und Konzentrationen nicht immer ausreichend. Darüber hinaus ist ihre fungitoxische Wirkung mit einer hohen Phytotoxizität verbunden, so daß bei den für die Bekämpfung von phytopathogenen Pilzen, beispielsweise bei der Bekämpfung von Mehltau, Schorf oder Rostpilzen notwendigen Konzentrationen auch wichtige Kulturpflanzen geschädigt oder in ihrem Wachstum stark gehemmt werden. Aus diesen Gründen sind sie zur Bekämpfung von phytopathogenen Pilzen nicht immer und nicht bei allen Kulturpflanzen geeignet.

Es wurde nun gefunden, daß Verbindungen der Formel I

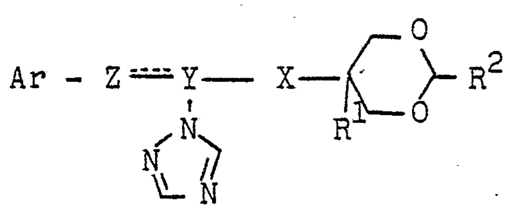

I

in der

$R^1$ und $R^2$ Wasserstoff, Alkyl mit 1 bis 5 C-Atomen oder Phenyl bedeuten, wobei der Phenylrest durch Fluor, Chlor, Brom, Nitro, Trifluormethyl, weiterhin durch Alkyl, Alkoxy oder Alkenyl mit jeweils 1 bis 3 C-Atomen sein kann und

WK/P

Ar Furanyl, Thienyl, Biphenylyl, Naphthyl oder Phenyl bedeutet, wobei der Phenylrest durch Fluor, Chlor, Brom, Iod, Nitro, Trifluormethyl, weiterhin durch Alkyl, Alkoxy oder Alkenyl mit jeweils 1 bis 5 C--Atomen und ferner durch Phenoxy substituiert sein kann und

Z⎓Y die Bedeutung CH=C oder $CH_2$-CH haben und

X eine -CO-, -CH(OH)- oder -CH($OR^3$)-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet - wobei der Benzylrest durch Fluor, Chlor, Brom, Nitro, Trifluormethyl; ferner durch Alkyl oder Alkoxy mit 1 bis 3 C-Atomen substituiert sein kann - oder $R^3$ einen -CO-$R^4$-Rest bedeutet, wobei $R^4$ einen gegebenenfalls durch Halogen, Alkoxy, Oxo (=O) oder Carboxyalkylsubstituierten Alkylrest mit 1 bis 5 C-Atomen oder einen aromatischen Rest bedeutet, sowie deren Salze und Metallkomplexverbindungen ausgezeichnet wirksam gegen Schadpilze, insbesondere aus der Klasse der Ascomyceten mit Basidiomyceten sind und eine sehr gute Pflanzenverträglichkeit zeigen.

Die neuen Verbindungen der Formel I enthalten chirale Zentren und/oder Doppelbindungen und werden im allgemeinen in Form von Racematen oder als Diastereomerengemische bzw. als Z- und E-Isomerengemische erhalten. Die Diastereomeren und E- und Z-Isomeren lassen sich bei einigen der erfindungsgemäßen Verbindungen beispielsweise durch Säulenchromatographie trennen oder aufgrund von Löslichkeitsunterschieden in reiner Form isolieren. Aus einheitlichen Diastereomeren kann man ferner mit bekannten Methoden einheitliche Enantiomeren erhalten. Diese werden von der vorliegenden Erfindung ebenfalls umfaßt. Für die Anwendung der erfindungsgemäßen Verbindungen als Fungizide sind sowohl die einheitlichen Diastereomeren, Enantiomeren bzw. geometrische Isomeren Z und E wie auch deren bei der Synthese anfallenden Gemische geeignet. Bevorzugt werden die letzteren verwendet.

$R^1$ und $R^2$ bedeuten beispielsweise Wasserstoff, Methyl, Ethyl, n-Propyl, Isopropyl, n-Butyl, sek.-Butyl, iso-Butyl, tert.-Butyl, n-Pentyl oder einen durch Fluor, Chlor, Brom, Methyl, Ethyl, Trifluormethyl, Methoxy, Ethoxy und Nitro substituierten Phenylrest.

Ar bedeutet beispielsweise 2-Furanyl, 2- und 3-Thienyl, 4-Biphenylyl, 1- und 2-Naphthyl, Phenyl, 4-Fluorphenyl, 2-Chlorphenyl, 3-Chlorphenyl, 4-Chlorphenyl, 4-Bromphenyl, 2,4-Dichlorphenyl, 3,4-Dichlorphenyl, 3,5-Dichlor-2-methoxyphenyl, 2,3,4-Trichlorphenyl, 2-Methoxyphenyl, 2,4-Dimethoxyphenyl, 3-Methoxyphenyl, 4-Methoxyphenyl, 4-Ethoxyphenyl, 4-tert.-Butoxyphenyl, 4-Methylphenyl, 4-Ethylphenyl, 4-Isopropylphenyl, 4-tert.-Butylphenyl, 3-Nitrophenyl, 4-Nitrophenyl, 3-Trifluormethylphenyl, 4-Trifluormethylphenyl und 4-Phenoxyphenyl.

X bedeutet beispielsweise eine Carbonylgruppe (C=O) oder die daraus durch Reduktion resultierenden Alkohole (-CHOH), die man weiterhin verethern (zu $-CH-OR^3$) oder verestern (zu $-CH-OCOR^4$) kann. Dabei bedeutet beispielsweise $R^3$ Methyl, Ethyl, n-Propyl, n-Butyl, 4-Chlorbutyl, n-Pentyl, n-Hexyl, Allyl, Buten-2-yl, Propargyl, Benzyl, 4-Fluorbenzyl, 4-Chlorbenzyl, 4-Brombenzyl, 2,4-Dichlorbenzyl und 4-Trifluormethylbenzyl. $R^4$ bedeutet vorzugsweise Methyl, Ethyl, n-Propyl, Isopropyl, Chlormethyl, Chlorpropyl, Methoxymethyl, Vinyl und Propenyl.

Die Verbindungen lassen sich herstellen, indem man

a) 1,2,4-Triazol mit α-Bromketonen der Formel II

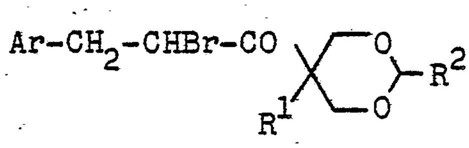 II,

worin $R^1$, $R^2$ und Ar die oben angegebene Bedeutung haben oder

b) ein Arylmethylhalogenid der Formel III

$$AR-CH_2-Y \qquad III,$$

worin Ar die oben genannte Bedeutung hat und Y Chlor oder Brom ist, mit einem 1-(1,3-Dioxan-5-yl)-2-(1,2,4-triazolyl-(s))-ethan-1-on der Formel IV

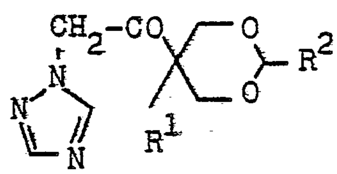 IV

worin $R^1$ und $R^2$ die oben genannte Bedeutung haben, oder

c) einen Aldehyd der Formel V

$$Ar - CH = O \qquad V,$$

in der Ar die oben genannte Bedeutung hat, mit dem 1-(1,3-Dioxan-5-yl)-2-(1,2,4-triazolyl-(1))-ethan-1-on-Derivat der Formel IV umsetzt

und die so erhaltenen Verbindungen gegebenenfalls anschließend reduziert und - falls gewünscht - danach verethert oder verestert.

Die Reaktion a) erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C. Zu den bevorzugten Lösungs- und Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, die gegebenenfalls auch als säurebindende Mittel bei der Reaktion verwendet werden können, sind beispielsweise Alkalihydroxide wie Lithium, Natrium- oder Kaliumhydroxid; Alkalicarbonate wie Natrium- oder Kaliumcarbonat oder Natrium- und Kaliumhydrogencarbonat, Überschüsse an 1,2,4-Triazol, Pyridin oder 4-Dimethylamino--pyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyl-triethylammonium-chlorid oder -bromid oder Kronenether wie 12-Krone-4, 15-Krone-5, 18-Krone-6, Dibenzo-18-Krone-6 oder Dicyclohexano-18-Krone-6 in Frage.

Die Reaktion b) erfolgt gegebenenfalls in Gegenwart eines Lösungs- oder Verdünnungsmittels und gegebenenfalls unter

Zusatz einer starken anorganischen oder organischen Base bei Temperaturen zwischen -10 und 120°C. Zu den bevorzugten Lösungs- oder Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride, wie Lithium-, Natrium- und Kaliumhydrid, Alkaliamide wie Natrium- und Kaliumamid, ferner Natrium- oder Kalium.-tert.-butoxid, Lithium-, Natrium- oder Kalium-Triphenylmethyl und Naphthalin-Lithium, -Natrium oder -Kalium.

Die Aldehyde V werden mit den Verbindungen IV gegebenenfalls in Gegenwart eines Lösungs- oder Verdünnungsmittels und gegebenenfalls unter Zusatz einer anorganischen oder organischen Base und/oder Säure bei Temperaturen zwischen -10 und 150°C gegebenenfalls unter azeotropischer Auskreisung des Reaktionswassers kondensiert. Zu den bevorzugten Lösungs- und Verdünnungsmitteln gehören Wasser, Alkohole wie Methanol, Ethanol, Isopropanol und tert.--Butanol, Kohlenwasserstoffe wie n-Hexan, n-Heptan, n- und iso-Octan, Cyclohexan, Tetrahydronaphthalin, Decahydronaphthalin, Toluol, Xylol, Cumol; Amide wie Dimethylformamid, Dimethylacetamid, N-Methylpyrrolidon und Ether wie Diethylether, Methyl-tert.-butylether, Dioxan, Tetrahydrofuran oder entsprechende Gemische.

Die Umsetzungen werden im allgemeinen bei Temperaturen zwischen 20 und 150°C, drucklos oder unter Druck, kontinuierlich oder diskontinuierlich durchgeführt.

Die 1-(1,3-Dioxan-5-yl)-2-(1,2,4-triazolyl-(1))-ethan-1-one der Formel IV sind neu. Sie können beispielsweise durch Umsetzung von 1-(1,3-Dioxan-5-yl)-2-halogen-ethan-1-onen der Formel VI

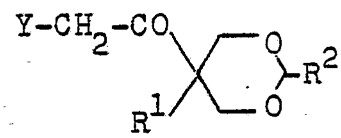 VI,

in welcher $R^1$ und $R^2$ die oben angegebenen Bedeutungen haben und Y Chlor oder Brom bedeutet, mit dem Natriumsalz des 1,2,4-Triazols erhalten werden.

Die Verbindungen der Formel VI sind ebenfalls neu. Man erhält sie z.B. durch Umsetzung von bekannten (1,3-Dioxan-5-yl)-ethan-1-onen der Formel VII

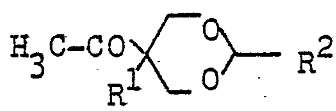 VII

in welcher $R^1$ und $R^2$ die oben angegebenen Bedeutungen haben, beispielsweise mit dem Pyrrolidon-Brom-Komplex nach D.P. Wyman u. P.R. Kaufman, J. Org. Chem. 29, 1956 (1964).

Die gemäß a), b) oder c) erhaltenen Ketone lassen sich gegebenenfalls in Gegenwart eines Lösungs- oder Verdünnungsmittels, bei Temperaturen zwischen -20 bis 150°C, bei Normaldruck oder unter erhöhtem Druck mit Wasserstoff in Gegenwart eines Katalysators, mit komplexen Bor- bzw. Aluminiumhydriden, mit Aluminiumisopropoxid und Isopropanol, mit Natriumdithionit oder elektrochemisch reduzieren.

Geeignete Lösungs- oder Verdünnungsmittel hierfür sind beispielsweise Wasser, Methanol, Ethanol, Isopropanol, Essigsäure, Diethylether, Tetrahydrofuran, Dioxan, Essigester, Toluol, Dimethylformamid oder entsprechende Gemische.

Zur katalytischen Hydrierung verwendet man Platin- oder Palladiumkatalysatoren auf inerten Trägern und hydriert bei Drucken von 2 bis 80 bar bis keine Wasserstoffaufnahme mehr erfolgt.

Für die als Reduktionsmittel verwendbaren Hydride seien beispielsweise Natriumborhydrid oder Lithiumaluminiumhydrid genannt.

Die so erhaltenen Alkohole der Formel I (X = CHOH) lassen sich mit Alkylierungsmitteln der Formel VIII

$$Z-R^3 \qquad VIII,$$

worin $R^3$ die oben angegebene Bedeutung hat und Z Chlor oder Brom darstellt, gegebenenfalls in Gegenwart eines Lösungs- oder Verdünnungsmittels die Ein- oder Zweiphasensysteme bilden, gegebenenfalls unter Zusatz einer anorganischen Base und gegebenenfalls unter Zusatz eines Reaktionsbeschleunigers und/oder Phasentransferkatalysators verethern.

Hierfür eignen sich folgende Lösungs- oder Verdünnungsmittel: Diethylether, Tetrahydrofuran, Dioxan, n-Pentan, monohalogenierte Kohlenwasserstoffe mit 2 bis 6 C-Atomen wie beispielsweise Chlorethan, Brommethan, 1-Chlorpropan, 1-Brompropan, 1-Chlorbutan, 1-Brombutan, 1-Chlorpentan, 1-Brompentan, 1-Chlorhexan, 1-Bromhexan, ferner Cyclohexan, Methylenchlorid, Chloroform, Toluol oder Dimethylformamid.

Als anorganische Basen seien beispielsweise genannt: Alkali- und Erdalkalihydroxide wie Natriumhydroxid, Kaliumhydroxid, Calciumhydroxid, Bariumhydroxid, Alkali- und Erdalkalicarbonate wie Natriumcarbonat, Kaliumcarbonat, Natriumhydrogencarbonat, Kaliumhydrogencarbonat, Calciumcarbonat, Bariumcarbonat, Alkali- oder Erdalkalialkoholate wie Natriummethylat, Natriumethylat, Magnesiummethylat, Natriumisopropylat oder Kalium-tert.-butylat.

Geeignete Reaktionsbeschleuniger sind beispielsweise Metallhalogenide wie Natriumbromid, Natriumiodid, Kaliumbromid, Kaliumiodid, tertiäre Amine wie 4-Dimethylaminopyridin und 4-Pyrrolidinopyridin, Kronenether wie 12-Krone-4-, 14-Krone-5, 18-Krone-6, Dibenzo-18-Krone-6 oder Dicyclohexano-18-Krone-6 oder Azole wie Imidazol und 1,2,4-Triazol.

Als Phasentransferkatalysatoren kommen vorzugsweise quaternäre Ammoniumsalze wie Tetrabutylammoniumchlorid, -bromid, -iodid oder -hydrogensulfat, Benzyl-triethylammoniumchlorid, Methyl-trioctylammoniumchlorid und -bromid und Phosphoniumsalze wie Tetrabutylphosphoniumbromid und -iodid und Tetra-n-pentylphosphoniumbromid und -iodid in Frage.

Die Veresterung der Alkohole der Formel I (X = CHOH) kann mit Säurechloriden oder -anhydriden der Formeln Y-CO-$R^4$ (IX) oder $(R^4\text{-CO})_2O$ (X) gegebenenfalls in Gegenwart eines Lösungs- oder Verdünnungsmittels, gegebenenfalls unter Zusatz eines säurebindenden Mittels und gegebenenfalls unter Zusatz eines Reaktionsbeschleunigers erfolgen. Hierfür eignen sich dieselben Lösungsmittel und Basen wie für die Veretherung. Zusätzlich können tertiäre Amine wie

Trimethylamin, Triethylamin, N,N-Dimethylanilin, N,N-Dimethylcyclohexylamin, N-Methylpiperidin oder Pyridin als Base verwendet werden.

Mit geeigneten Basen wie z.B. Alkalihydrid wie Natriumhydrid oder Alkali- oder Erdalkalialkoholaten wie Natriummethylat können die Alkohole auch in einer ersten Umsetzung zunächst in ihre Alkoholat-Salze überführt werden und dann als solche zur Reaktion gebracht werden.

Die so erhaltenen Verbindungen der Formel I werden nach üblichen Methoden isoliert, gegebenenfalls gereinigt und gegebenenfalls mit Säuren oder Metallsalzen zu Salzen bzw. zu Metall-Komplexen umgesetzt.

Als Beispiele für die neuen Verbindungen der Formel I seien im einzelnen genannt:

1-(5-Methyl-1,3-dioxan-5-yl)-2-(1,2,4-triazolyl-(1))-3--(2-furanyl)-propen-1-on,
1-(5-Methyl-1,3-dioxan-5-yl)-2-(1,2,4-triazolyl-(1))-3--(2-furanyl)-propen-1-ol,
1-(5-Methyl-1,3-dioxan-5-yl)-2-(1,2,4-triazolyl-(1))-3--(2-furanyl)-propen-2,
1-(5-Methyl-1,3-dioxan-5-yl)-2-(1,2,4-triazolyl-(1))-3--phenyl-propan-1-on,
1-(5-Methyl-1,3-dioxan-5-yl)-2-(1,2,4-triazolyl-(1))-3--phenyl-propan-1-ol,
1-(5-Methyl-1,3-dioxan-5-yl)-1-methoxy-2-(1,2,4-triazolyl-(1))-3-phenyl-propan,
1-(5-Methyl-1,3-dioxan-5-yl)-1-ethoxy-2-(1,2,4-triazolyl-(1))-3-phenyl-propan,
1-(2-(4'-Methylphenyl)-5-methyl-1,3-dioxan-5-yl)-2--(1,2,4-triazolyl-(1))-3-phenyl-propan-1-on

1-(2-(4'-Methoxyphenyl)-5-methyl-1,3-dioxan-5-yl)-2--(1,2,4-triazolyl-(1))-3-phenyl-propan-1-on,
1-(2-(4'-Nitrophenyl)-5-methyl-1,3-dioxan-5-yl)-2-(1,2,4--triazolyl-(1))-3-phenyl-propan-1-on,
1-(5-Methyl-1,3-dioxan-5-yl)-1-n-pentyloxy-2-(1,2,4-triazolyl-(1))-3-phenyl-propan,
1-(5-Methyl-1,3-dioxan-5-yl)-1-allyloxy-2-(1,2,4-triazolyl-(1))-3-phenyl-propan,
1-(5-Methyl-1,3-dioxan-5-yl)-1-propoargyloxy-2-(1,2,4--triazolyl-(1))-3-phenyl-propan,
1-(5-Methyl-1,3-dioxan-5-yl)-2-(1,2,4-Triazolyl-(1))-3--phenyl-propen-1-on,
1-(5-Methyl-1,3-dioxan-5-yl)-2-(1,2,4-triazolyl-(1))-3--phenyl-propen-1-ol,
1-(2-Ethyl-5-methyl-1,3-dioxan-5-yl)-2-(1,2,4-triazolyl-(1))-3-phenyl-propan-1-on,
1-(2-Ethyl-5-methyl-1,3-dioxan-5-yl)-2-(1,2,4-triazolyl-(1))-3-phenyl-propan-1-ol,
1-(2-Ethyl-5-methyl-1,3-dioxan-5-yl)-2-(1,2,4-triazolyl--(1))-3-phenyl-propen-1-ol,
1-(2-Ethyl-5-methyl-1,3-dioxan-5-yl)-2-(1,2,4-triazolyl--(1))-3-phenyl-propen-1-on,
1-(2-n-Propyl-5-methyl-1,3-dioxan-5-yl)-2-(1,2,4-triazolyl-(1))-3-phenyl-propen-1-on,
1-(2-n-Propyl-5-methyl-1,3-dioxan-5-yl)-2-(1,2,4-triazolyl-(1))-3-phenyl-propen-1-ol,
1-(5-Methyl-1,3-dioxan-5-yl)-1-acetoxy-(1,2,4-triazolyl--(1))-3-phenyl-propen-2,
1-(5-Methyl-1,3-dioxan-5-yl)-1-propionyloxy-(1,2,4-triazolyl-(1))-3-phenyl-propen-2,
1-(2-Ethyl-5-methyl-1,3-dioxan-5-yl)-1-propionyloxy--(1,2,4-triazolyl-(1))-3-phenyl-propen-2,
1-(2-Ethyl-5-methyl-1,3-dioxan-5-yl)-2-benzoyloxy--(1,2,4-triazolyl-(1))-3-phenyl-propen-2,

1-(5-Methyl-1,3-dioxan-5-yl)-2-(1,2,4-triazolyl-(1))-3-(1-naphthyl)-propan-1-on,
1-(5-Methyl-1,3-dioxan-5-yl)-2-(1,2,4-triazolyl-(1))-3-(1-naphthyl)-propan-1-ol,
1-(5-Methyl-1,3-dioxan-5-yl)-2-(1,2,4-triazolyl-(1))-3-(1-naphthyl)-propen-1-on,
1-(5-Methyl-1,3-dioxan-5-yl)-2-(1,2,4-triazolyl-(1))-3-(1-naphthyl)-propen-1-ol,
1-(2-Ethyl-5-methyl-1,3-dioxan-5-yl)-2-(1,2,4-triazolyl-(1))-3-(1-naphthyl)-propen-1-on,
1-(2-Ethyl-5-methyl-1,3-dioxan-5-yl)-2-(1,2,4-triazolyl-(1))-3-(1-naphthyl)-propen-1-ol,
1-(2,5-Diethyl-1,3-dioxan-5-yl)-2-(1,2,4-triazolyl-(1))-3-(1-naphthyl)-propen-1-on,
1-(2,5-Diethyl-1,3-dioxan-5-yl)-2-(1,2,4-triazolyl-(1))-3-(1-naphthyl)-propen-1-ol,
1-(2-Ethyl-5-methyl-1,3-dioxan-5-yl)-1-acetoxy-(1,2,4-triazolyl-(1))-3-(1-naphthyl)-propen-2,
1-(5-Methyl-1,3-dioxan-5-yl)-2-(1,2,4-triazolyl-(1))-3-(4-biphenylyl)-propen-1-on,
1-(5-Methyl-1,3-dioxan-5-yl)-2-(1,2,4-triazolyl-(1))-3-(4-biphenylyl-propen-1-ol,
1-(2-Ethyl-5-methyl-1,3-dioxan-5-yl)-2-(1,2,4-triazolyl-(1))-3-(1-naphthyl)-propan-1-on,
1-(2-Ethyl-5-methyl-1,3-dioxan-5-yl)-2-(1,2,4-triazolyl-(1))-3-(1-naphthyl)-propan-1-ol,
1-(2-Propyl-5-methyl-1,3-dioxan-5-yl)-2-(1,2,4-triazolyl-(1))-3-(1-naphthyl)-propan-1-on,
1-(2-Ethyl-5-methyl-1,3-dioxan-5-yl)-1-acetoxy-2-(1,2,4-triazolyl-(1))-3-(1-naphthyl)-propan,
1-(5-Methyl-1,3-dioxan-5-yl)-2-(1,2,4-triazolyl-(1))-3-(4-fluorphenyl)-propan-1-on,
1-(5-Methyl-1,3-dioxan-5-yl)-2-(1,2,4-triazolyl-(1))-3-(4-fluorphenyl)-propan-1-ol,

1-(5-Methyl-1,3-dioxan-5-yl)-2-(1,2,4-triazolyl-(1))-3-(4-fluorphenyl)-propen-1-on,
1-(5-Methyl-1,3-dioxan-5-yl)-2-(1,2,4-triazolyl-(1))-3-(4-fluorphenyl)-propen-1-ol,
1-(2-Ethyl-5-methyl-1,3-dioxan-5-yl)-2-(1,2,4-triazolyl-(1))-3-(4-fluorphenyl)-propan-1-on,
1-(2-Ethyl-5-methyl-1,3-dioxan-5-yl)-2-(1,2,4-triazolyl-(1))-3-(4-fluorphenyl)-propan-1-ol,
1-(5-Methyl-1,3-dioxan-5-yl)-2-(1,2,4-triazolyl-(1))-3-(4-chlorphenyl)-propan-1-on,
1-(5-Methyl-1,3-dioxan-5-yl)-2-(1,2,4-triazolyl-(1))-3-(2-chlorphenyl)-propen-1-ol,
1-(2-Ethylen-5-methyl-1,3-dioxan-5-yl)-2-(1,2,4-triazolyl-(1))-3-(2-chlorphenyl)-propen-1-on,
1-(2-Ethyl-5-methyl-1,3-dioxan-5-yl)-2-(1,2,4-triazolyl-(1))-3-(2-chlorphenyl)-propen-1-ol,
1-(2-Ethyl-5-methyl-1,3-dioxan-5-yl)-1-acetoxy-(1,2,4-triazolyl-(1))-3-(2-chlorphenyl)-propen-2,
1-(5-Methyl-1,3-dioxan-5-yl)-2-(1,2,4-triazolyl-(1))-3-(3-chlorphenyl)-propen-1-on,
1-(5-Methyl-1,3-dioxan-5-yl)-2-(1,2,4-triazolyl-(1))-3-(3-chlorphenyl)-propen-1-ol,
1-(5-Methyl-1,3-dioxan-5-yl)-1-propionyloxy-2-(1,2,4-triazolyl-(1))-3-(3-chlorphenyl)-propen-2,
1-(5-Methyl-1,3-dioxan-5-yl)-2-(1,2,4-triazolyl-(1))-3-(4-chlorphenyl)-propen-1-on,
1-(5-Methyl-1,3-dioxan-5-yl)-2-(1,2,4-triazolyl-(1))-3-(4-chlorphenyl)-propen-1-ol,
1-(5-Methyl-1,3-dioxan-5-yl)-2-(1,2,4-triazolyl-(1))-3-(4-chlorphenyl)-propan-1-ol,
1-(5-Methyl-1,3-dioxan-5-yl)-1-propionyloxy-2-(1,2,4-triazolyl-(1))-3-(4-fluorphenyl)-propen-2,
1-(2-Ethyl-5-methyl-1,3-dioxan-5-yl)-2-(1,2,4-triazolyl-(1))-3-(4-chlorphenyl)-propen-1-on,
1-(2-Ethyl-5-methyl-1,3-dioxan-5-yl)-2-(1,2,4-triazolyl-

-(1))-3-(4-chlorphenyl)-propen-1-ol-
1-(2-n-Propyl-5-methyl-1,3-dioxan-5-yl)-2-(1,2,4-triazolyl-(1))-3-(4-chlorphenyl)-propen-1-on,
1-(5-Methyl-1,3-dioxan-5-yl)-2-(1,2,4-triazolyl-(1))-3-(2-chlorphenyl)-propen-1-on,
1-(2,5-Diethyl-1,3-dioxan-5-yl)-2-(1,2,4-triazolyl-(1))-3-(4-chlorphenyl)-propen-1-ol,
1-(2,5-Diethyl-1,3-dioxan-5-yl)-2-(1,2,4-triazolyl-(1))-3-(4-chlorphenyl)-propen-1-on,
1-(2,5-Dimethyl-1,3-dioxan-5-yl)-2-(1,2,4-triazolyl-(1))-3-(4-chlorphenyl)-propan-1-on,
1-(2,5-Dimethyl-1,3-dioxan-5-yl)-2-(1,2,4-triazolyl-(1))-3-(4-chlorphenyl)-propan-1-ol,
1-(2-Ethyl-5-methyl-1,3-dioxan-5-yl)-2-(1,2,4-triazolyl-(1))-3-(4-chlorphenyl)-propan-1-on,
1-(2-Ethyl-5-methyl-1,3-dioxan-5-yl)-2-(1,2,4-triazolyl-(1))-3-(4-chlorphenyl)-propan-1-ol,
1-(2-n-Propyl-5-methyl-1,3-dioxan-5-yl)-2-(1,2,4-triazolyl-(1))-3-(4-chlorphenyl)-propan-1-on,
1-(2-n-Propyl-5-methyl-1,3-dioxan-5-yl)-2-(1,2,4-triazolyl-(1))-3-(4-chlorphenyl)-propan-1-ol,
1-(2-Isopropyl-5-methyl-1,3-dioxan-5-yl)-2-(1,2,4-triazolyl-(1))-3-(4-chlorphenyl)-propan-1-on,
1-(2-Isopropyl-5-methyl-1,3-dioxan-5-yl)-2-(1,2,4-triazolyl-(1))-3-(4-chlorphenyl)-propan-1-ol,
1-(2-n-Butyl-5-methyl-1,3-dioxan-5-yl)-2-(1,2,4-triazolyl-(1))-3-(4-chlorphenyl)-propan-1-on,
1-(2-n-Butyl-5-methyl-1,3-dioxan-5-yl)-2-(1,2,4-triazolyl-(1))-3-(4-chlorphenyl)-propan-1-ol,
1-(5-Methyl-1,3-dioxan-5-yl)-1-methoxy-2-(1,2,4-triazolyl-(1))-3-(4-chlorphenyl)-propan,
1-(5-Methyl-1,3-dioxan-5-yl)-1-(2-butenoyloxy)-2-(1,2,4-triazolyl-(1))-3-(4-chlorphenyl)-propan,
1-(5-Methyl-1,3-dioxan-5-yl)-1-n-propoxy-2-(1,2,4-triazolyl-(1)-3-(4-chlorphenyl)-propan,

1-(5-Methyl-1,3-dioxan-5-yl)-1-n-butoxy-2-(1,2,4-triazolyl-(1))-3-(4-chlorphenyl)-propan,
1-(5-Methyl-1,3-dioxan-5-yl)-1-acetoxy-2-(1,2,4-triazolyl-(1))-3-(4-chlorphenyl)-propan,
1-(2-Ethyl-5-methyl-1,3-dioxan-5-yl)-1-methoxy-2-(1,2,4--triazolyl-(1))-3-(4-chlorphenyl)-propan,
1-(2-Ethyl-5-methyl-1,3-dioxan-5-yl)-1-n-propoxy-2-(1,2,4--triazolyl-(1))-3-(4-chlorphenyl)-propan,
1-(2-Phenyl-5-methyl-1,3-dioxan-5-yl)-2-(1,2,4-triazolyl--(1))-3-(4-chlorphenyl)-propan-1-on,
1-(2-Phenyl-5-methyl-1,3-dioxan-5-yl)-2-(1,2,4-triazolyl--(1))-3-(4-chlorphenyl)-propan-1-ol,
1-(2-Phenyl-5-methyl-1,3-dioxan-5-yl)-1-propoxy-2-(1,2,4--triazolyl-(1))-3-(4-chlorphenyl)-propan,
1-(2-Ethyl-5-methyl-1,3-dioxan-5-yl)-1-acetoxy-2-(1,2,4--triazolyl-(1))-3-(4-chlorphenyl)-propan,
1-(2-Ethyl-5-methyl-1,3-dioxan-5-yl)-1-propionyloxy-2--(1,2,4-triazolyl-(1))-3-(4-chlorphenyl)-propan,
1-(2-Ethyl-5-methyl-1,3-dioxan-5-yl)-1-butyryloxy-2--(1,2,4-triazolyl-(1))-3-(4-chlorphenyl)-propan,
1-(5-Methyl-1,3-dioxan-5-yl)-2-(1,2,4-triazolyl--(1))-3-(4-bromphenyl)-propan-1-on,
1-(5-Methyl-1,3-dioxan-5-yl)-2-(1,2,4-triazolyl-(1))--3-(4-bromphenyl)-propan-1-ol,
1-(2-Ethyl-5-methyl-1,3-dioxan-5-yl)-2-(1,2,4-triazolyl--(1))-3-(4-bromphenyl)-propan-1-on,
1-(2-Ethyl-5-methyl-1,3-dioxan-5-yl)-2-(1,2,4-triazolyl)--(1))-3-(4-bromphenyl)-propan-1-ol,
1-(5-Methyl-1,3-dioxan-5-yl)-2-(1,2,4-triazolyl-(1))-3--(4-bromphenyl)-propen-1-on,
1-(5-Methyl-1,3-dioxan-5-yl)-2-(1,2,4-triazolyl-(1))-3--(4-bromphenyl)-propen-1-ol,
1-(5-Methyl-1,3-dioxan-5-yl)-2-(1,2,4-triazolyl-(1))-3--(2,4-dichlorphenyl)-propan-1-on,
1-(5-Methyl-1,3-dioxan-5-yl)-2-(1,2,4-triazolyl-(1))-3-

-(2,4-dichlorphenyl)-propan-1-ol,
1-(2-Ethyl-5-methyl-1,3-dioxan-5-yl)-2-(1,2,4-triazolyl--(1))-3-(2,4-dichlorphenyl)-propan-1-on,
1-(2-Ethyl-5-methyl-1,3-dioxan-5-yl)-2-(1,2,4-triazolyl--(1))-3-(2,4-dichlorphenyl)-propan-1-ol,
1-(2,5-Methyl-1,3-dioxan-5-yl)-2-(1,2,4-triazolyl-(1))-3--(2,4-dichlorphenyl)-propan-1-on,
1-(2,5-Diethyl-1,3-dioxan-5-yl)-2-(1,2,4-triazolyl-(1))-3--(2,4-dichlorphenyl)-propan-1-ol,
1-(2-n-Propyl-5-methyl-1,3-dioxan-5-yl)-2-(1,2,4-triazolyl--(1))-3-(2,4-dichlorphenyl)-propan-1-ol,
1-(2-n-Propyl-5-methyl-1,3-dioxan-5-yl)-2-(1,2,4-triazolyl--(1))-3-(2,4-dichlorphenyl)-propan-1-on,
1-(2-Phenyl-5-methyl-1,3-dioxan-5-yl)-2-(1,2,4-triazolyl--(1))-3-(2,4-dichlorphenyl)-propan-1-on,
1-(2-Phenyl-5-methyl-1,3-dioxan-5-yl)-2-(1,2,4-triazolyl--(1))-3-(2,4-dichlorphenyl)-propan-1-ol,
1-(2-(4'-Chlorphenyl)-5-methyl-1,3-dioxan-5-yl)-2-(1,2,4--triazolyl-(1))-3-(2,4-dichlorphenyl)-propan-1-on,
1-(5-Methyl-1,3-dioxan-5-yl)-2-(1,2,4-triazolyl-(1))-3--(3,4-dichlorphenyl)-propen-1-on,
1-(5-Methyl-1,3-dioxan-5-yl)-2-(1,2,4-triazolyl-(1))-3--(3,4-dichlorphenyl)-propan-1-ol,
1-(5-Methyl-1,3-dioxan-5-yl)-2-(1,2,4-triazolyl-(1))-3--(2,4-dichlorphenyl)-propen-1-on,
1-(5-Methyl-1,3-dioxan-5-yl)-2-(1,2,4-triazolyl-(1))-3--(2,4-dichlorphenyl)-propen-1-ol,
1-(2-Ethyl-5-methyl-1,3-dioxan-5-yl)-2-(1,2,4-triazolyl--(1))-3-(2,4-dichlorphenyl)-propen-1-on,
1-(2-Ethyl-5-methyl-1,3-dioxan-5-yl)-2-(1,2,4-triazolyl--(1))-3-(2,4-dichlorphenyl)-propen-1-ol,
1-(2,5-Diethyl-1,3-dioxan-5-yl)-2-(1,2,4-triazolyl-(1))--3-(2,4-dichlorphenyl)-propen-1-on,
1-(2,5-Diethyl-1,3-dioxan-5-yl)-2-(1,2,4-triazolyl-(1))--3-(2,4-dichlorphenyl)-propen-1-ol,

1-(2-Isopropyl-5-methyl-1,3-dioxan-5-yl)-2-(1,2,4-triazolyl-(1))-3-(2,4-dichlorphenyl)-propen-1-on,

1-(2-Isopropyl-5-methyl-1,3-dioxan-5-yl)-2-(1,2,4-triazolyl-(1))-3-(2,4-dichlorphenyl)-propen-1-ol,

1-(2-n-Butyl-5-methyl-1,3-dioxan-5-yl)-2-(1,2,4-triazolyl-(1))-3-(2,4-dichlorphenyl)-propen-1-on,

1-(2-n-Butyl-5-methyl-1,3-dioxan-5-yl)-2-(1,2,4-triazolyl-(1))-3-(2,4-dichlorphenyl)-propen-1-ol,

1-(5-Methyl-1,3-dioxan-5-yl)-1-acetoxy-2-(1,2,4-triazolyl-(1))-3-(2,4-dichlorphenyl)-propen-2,

1-(5-Methyl-1,3-dioxan-5-yl)-1-butyryloxy-2-(1,2,4-triazolyl-(1))-3-(2,4-dichlorphenyl)-propen-2,

1-(2-Ethyl-5-methyl-1,3-dioxan-5-yl)-1-acetoxy-2-(1,2,4-triazolyl-(1))-3-(2,4-dichlorphenyl)-propen-2,

1-(2-Ethyl-5-methyl-1,3-dioxan-5-yl)-1-propionyloxy-2-(1,2,4-triazolyl-(1))-3-(2,4-dichlorphenyl)-propen-2,

1-(5-Methyl-1,3-dioxan-5-yl)-2-(1,2,4-triazolyl-(1))-3-(2,3,4-trichlorphenyl)-propen-1-on,

1-(5-Methyl-1,3-dioxan-5-yl)-2-(1,2,4-triazolyl-(1))-3-(2,3,4-trichlorphenyl)-propen-1-ol,

1-(2-Isopropyl-5-methyl-1,3-dioxan-5-yl)-2-(1,2,4-triazolyl-(1))-3-(2,4-dichlorphenyl)-propan-1-on,

1-(2-Isopropyl-5-methyl-1,3-dioxan-5-yl)-2-(1,2,4-triazolyl-(1))-3-(2,4-dichlorphenyl)-propan-1-ol,

1-(2-n-Butyl-5-methyl-1,3-dioxan-5-yl)-2-(1,2,4-triazolyl-(1)-3-(2,4-dichlorphenyl)-propan-1-on,

1-(2-n-Butyl-5-methyl-1,3-dioxan-5-yl)-2-(1,2,4-triazolyl-(1))-3-(2,4-dichlorphenyl)-propan-1-ol,

1-(2-<u>tert.</u>-Butyl-5-methyl-1,3-dioxan-5-yl)-2-(1,2,4-triazolyl-(1))-3-(2,4-dichlorphenyl)-propan-1-on,

1-(2-<u>tert.</u>-Butyl-5-methyl-1,3-dioxan-5-yl)-2-(1,2,4-triazolyl-(1))-3-(2,4-dichlorphenyl)-propan-1-ol,

1-(5-Methyl-1,3-dioxan-5-yl)-1-acetoxy-2-(1,2,4-triazolyl-(1))-3-(2,4-dichlorphenyl)-propan,

1-(5-Methyl-1,3-dioxan-5-yl)-1-propienyloxy-2-(1,2,4-triazolyl-(1))-3-(2,4-dichlorphenyl)-propan,
1-(2-Ethyl-5-methyl-1,3-dioxan-5-yl)-1-acetoxy-2-(1,2,4-triazolyl-(1))-3-(2,4-dichlorphenyl)-propan,
1-(2-Isopropyl-5-methyl-1,3-dioxan-5-yl)-1-acetoxy-2-(1,2,4-triazolyl-(1))-3-(2,4-dichlorphenyl)-propan,
1-(5-Methyl-1,3-dioxan-5-yl)-2-(1,2,4-triazolyl-(1))-3-(3,4-dichlorphenyl)-propan-1-on,
1-(5-Methyl-1,3-dioxan-5-yl)-2-(1,2,4-triazolyl-(1))-3-(3,4-dichlorphenyl)-propan-1-ol,
1-(2-Ethyl-5-methyl-1,3-dioxan-5-yl)-2-(1,2,4-triazolyl-(1))-3-(3,4-dichlorphenyl)-propan-1-ol,
1-(2-Ethyl-5-methyl-1,2-dioxan-5-yl)-2-(1,2,4-triazolyl-(1))-3-(3,4-dichlorphenyl)-propan-1-on,
1-(2-Ethyl-5-methyl-1,2-dioxan-5-yl)-1-acetoacetoxy-2-(1,2,4-triazolyl-(1))-3-(3,4-dichlorphenyl)-propan,
1-(2-Ethyl-5-propyl-1,2-dioxan-5-yl)-1-benzoyl-2-(1,2,4-triazolyl-(1))-3-(3,4-dichlorphenyl)-propan,
1-(5-Methyl-1,2-dioxan-5-yl)-2-(1,2,4-triazolyl-(1))-3-(4-methylphenyl)-propan-1-on,
1-(5-Methyl-1,3-dioxan-5-yl)-2-(1,2,4-triazolyl-(1))-3-(4-methylphenyl)-propen-1-on,
1-(5-Methyl-1,3-dioxan-5-yl)-2-(1,2,4-triazolyl-(1))-3-(4-methylphenyl)-propen-1-ol,
1-(5-Methyl-1,3-dioxan-5-yl)-2-(1,2,4-triazolyl-(1))-3-(4-methylphenyl)-propan-1-ol,
1-(2-Ethyl-5-methyl-1,3-dioxan-5-yl)-2-(1,2,4-triazolyl-(1))-3-(4-methylphenyl)-propan-1-on,
1-(2-Ethyl-5-methyl-1,3-dioxan-5-yl)-2-(1,2,4-triazolyl-(1))-3-(4-methylphenyl)-propan-1-ol,
1-(5-Methyl-1,3-dioxan-5-yl)-2-(1,2,4-triazolyl-(1))-3-(3-methylphenyl)-propan-1-on,
1-(5-Methyl-1,3-dioxan-5-yl)-2-(1,2,4-triazolyl-(1))-3-(3-methylphenyl)-propan-1-ol,
1-(5-Methyl-1,3-dioxan-5-yl)-2-(1,2,4-triazolyl-(1))-3-(4-ethylphenyl)-propan-1-on,

1-(5-Methyl-1,3-dioxan-5-yl)-2-(1,2,4-triazolyl-(1))-3--(4-ethylphenyl)-propan-1-ol,
1-(5-Methyl-1,3-dioxan-5-yl)-2-(1,2,4-triazolyl-(1))-3--(3,4-dimethylphenyl)-propan-1-on,
1-(5-Methyl-1,3-dioxan-5-yl)-2-(1,2,4-triazolyl-(1))-3--(3,4-dimethylphenyl)-propan-1-ol,
1-(2-Ethyl-5-methyl-1,3-dioxan-5-yl)-2-(1,2,4-triazolyl--(1))-3-(4-methoxyphenyl)-propan-1-on,
1-(2-Ethyl-5-methyl-1,3-dioxan-5-yl)-2-(1,2,4-triazolyl--(1))-3-(4-methoxyphenyl)-propan-1-ol,
1-(2-Ethyl-5-methyl-1,3-dioxan-5-yl)-2-(1,2,4-triazolyl--(1))-3-(4-nitrophenyl)-propan-1-on,
1-(5-Methyl-1,3-dioxan-5-yl)-2-(1,2,4-triazolyl-(1))-3--(4-trifluormethylphenyl)-propan-1-on,
1-(5-Methyl-1,3-dioxan-5-yl)-2-(1,2,4-triazolyl-(1))-3ß--(4-trifluormethylphenyl)-propan-1-ol,
1-(2-Ethyl-5-methyl-1,3-dioxan-5-yl)-2-(1,2,4-triazolyl--(1))-3-(4-trifluormethylphenyl)-propan-1-on,
1-(2-Ethyl-5-methyl-1,3-dioxan-5-yl)-2-(1,2,4-triazolyl--(1))-3-(4-trifluormethylphenyl)-propan-1-ol,
1-(2-Ethyl-5-methyl-1,3-dioxan-5-yl)-2-(1,2,4-triazolyl--(1))-3-(4-tert.-Butylphenyl)-propan-1-on,
1-(5-Methyl-1,3-dioxan-5-yl)-2-(1,2,4-triazolyl-(1))-3--(4-tert.-butylphenyl)-propen-1-ol,
1-(5-Methyl-1,3-dioxan-5-yl)-2-(1,2,4-triazolyl-(1))-3--(4-methoxyphenyl)-propen-1-ol,
1-(5-Methyl-1,3-dioxan-5-yl)-2-(1,2,4-triazolyl-(1))-3--(4-methoxyphenyl)-propen-1-on,
1-(2-Ethyl-5-methyl-1,3-dioxan-5-yl)-2-(1,2,4-triazolyl--(1))-3-(4-methoxyphenyl)-propen-1-on,
1-(2-Ethyl-5-methyl-1,3-dioxan-5-yl)-2-(1,2,4-triazolyl--(1))-3-(4-methoxyphenyl)-propen-1-ol,
1-(5-Methyl-1,3-dioxan-5-yl)-2-(1,2,4-triazolyl-(1))-3--(4-ethoxyphenyl)-propen-1-on,
1-(5-Methyl-1,3-dioxan-5-yl)-2-(1,2,4-triazolyl-(1))-3-

-(4-ethoxyphenyl)-propen-1-ol,
1-(5-Methyl-1,3-dioxan-5-yl)-2-(1,2,4-triazolyl-(1))-3-
-(4-n-butoxyphenyl)-propen-1-on,
1-(5-Methyl-1,3-dioxan-5-yl)-2-(1,2,4-triazolyl-(1))-3-
-(4-n-butoxyphenyl)-propen-1-ol,
1-(5-Methyl-1,3-dioxan-5-yl)-2-(1,2,4-triazolyl-(1))-3-
-(4-tert.-butoxyphenyl)-propen-1-ol,
1-(5-Methyl-1,3-dioxan-5-yl)-2-(1,2,4-triazolyl-(1))-3-
-(4-tert.-butoxyphenyl)-propen-1-on,
1-(5-Methyl-1,3-dioxan-5-yl)-2-(1,2,4-triazolyl-(1))-3-
-(4-nitrophenyl)-propen-1-on,
1-(5-Methyl-1,3-dioxan-5-yl)-2-(1,2,4-triazolyl-(1))-3-
-(4-nitrophenyl)-propen-1-ol,
1-(5-Methyl-1,3-dioxan-5-yl)-2-(1,2,4-triazolyl-(1))-3-
-(3-trifluormethylphenyl)-propen-1-on,
1-(5-Methyl-1,3-dioxan-5-yl)-2-(1,2,4-triazolyl-(1))-3-
-(3-trifluormethylphenyl)-propen-1-ol,
1-(5-Methyl-1,3-dioxan-5-yl)-1-propionyloxy-2-(1,2,4-triazolyl-(1))-3-(3-trifluormethylphenyl)-propen-2,
1-(5-Methyl-1,3-dioxan-5-yl)-2-(1,2,4-triazolyl-(1))-3-
-(4-trifluormethylphenyl)-propen-1-on,
1-(5-Methyl-1,3-dioxan-5-yl)-2-(1,2,4-triazolyl-(1))-3-
-(4-trifluormethylphenyl)-propen-1-ol,
1-(2-Ethyl-5-methyl-1,3-dioxan-5-yl)-2-(1,2,4-triazolyl-
-(1))-3-(4-trifluormethylphenyl)-propen-1-on,
1-(2-Ethyl-5-methyl-1,3-dioxan-5-yl)-2-(1,2,4-triazolyl-
-(1))-3-(4-trifluormethylphenyl)-propen-1-ol,
1-(5-Methyl-1,3-dioxan-5-yl)-2-(1,2,4-triazolyl-(1))-3-
-(4-ethoxyphenyl)-propan-1-on,
1-(5-Methyl-1,3-dioxan-5-yl)-2-(1,2,4-triazolyl-(1))-3-
-(4-ethoxyphenyl)-propan-1-on,

Die folgenden Beispiele erläutern die Herstellung der Substanzen:

Beispiel 1

a) Herstellung des Ausgangsmaterials

Zu einer Lösung von 144 g (1 Mol) 5-Acetyl-5-methyl-1,3-dioxan und 85,5 g (1 Mol) Pyrrolidon in 500 ml Tetrahydrofuran wird in 2 Stunden bei 50°C die Lösung von 498 g (1 Mol) Pyrrolidon-Brom-Komplex in 1 l Tetrahydrofuran zugetropft. Nach achtstündigem Nachrühren bei 50°C wird der weiße Niederschlag von Pyrrolidon-Hydrobromid abgesaugt, mit 50 ml Tetrahydrofuran gewaschen und das Filtrat im Vakuum eingeengt. Man erhält 220 g (99 %) rohes, öliges 1-(5-Methyl-1,3-dioxan-5-yl)-2-brom-ethan-1-on.

Zu einer unter reinem Stickstoff gerührten Suspension von 100,1 g (1,1 Mol) Natrium-1,2,4-triazolid in 300 ml trockenem Tetrahydrofuran wird die Lösung von 223 g (1 Mol (1-(5-Methyl-1,3-dioxan-5-yl)-2-brom-ethan-1-on in 200 ml Tetrahydrofuran bei 25°C in 2 Stunden zugetropft. Nach achtstündigem Erhitzen unter Rückfluß wird der anorganische Niederschlag abfiltriert und das Filtrat zur Hälfte eingeengt. Das Gemisch wird angeimpft und über Nacht bei +3°C stehen gelassen. Der Niederschlag wird abgesaugt, mit 30 ml kaltem (+5°C) Tetrahydrofuran, dann mit 80 ml Ether und anschließend mit 100 ml n-Pentan gewaschen und getrocknet. Man erhält 184 g (87,2 %) 1-(5-Methyl-1,3-dioxan-5-yl)-2-(1,2,4-triazolyl-(1))-ethan-1-on als weiße Kristalle vom Schmelzpunkt 95 bis 97°C.

b) Herstellung des Endproduktes

Zu einer unter reinem Stickstoff gerührten Suspension von 13,2 g (0,55 Mol) Natriumhydrid in 100 ml trockenem Dimethylformamid wird die Lösung von 105,5 (0,5 Mol) 1-(5-Methyl-1,3-dioxan-5-yl)-2-(1,2,4-triazolyl-(1))-ethan-

-1-on in 100 ml Dimethylformamid bei 20 bis 25°C zugetropft. Nach dreistündigem Nachrühren wird bei 25°C die Lösung von 81 g (0,5 Mol) 4-Chlorbenzylchlorid in 50 ml Dimethylformamid zugetropft und das Reaktionsgemisch weitere 14 Stunden nachgerührt. 50 ml Eiswasser werden vorsichtig zugetropft und das Gemisch wird im Vakuum eingeengt. Der Rückstand wird zwischen 400 ml Methylenchlorid und 200 ml Wasser verteilt, die organische Phase dreimal mit je 200 ml Wasser gewaschen, über $Na_2SO_2$ getrocknet und eingeengt. Man erhält 112 g (66,8 %) 1-(5-Methyl-1,3-dioxan-5-yl)-2-(1,2,4-triazolyl-(1))-3-(4-chlorphenyl)-propan-1-on als blaßgelbes Harz.

$^1H$-NMR (80 MHz/$CDCl_3$): = 0,87 (s, 3H), 3,1-3,6 (m, 4H), 3,95-4,4 (t, 2H), 4,5-5,0 (2 dd, zus. 2H), 5,65-5,95 (q, 1H), 6,8-7,3 (m, 4H), 7,8 (s 1H), 8,0 ppm (s, 1H).

Beispiel 2

Zu einer Lösung von 90 g (0,269 Mol) 1-(5-Methyl-1,3-dioxan-5-yl)-2-(1,2,4-triazolyl-(1))-3-(4-chlorphenyl)-propan-1-on in 250 ml Methanol werden zwischen 0 und +5°C 11,5 g (0,3 Mol) Natriumborhydrid portionsweise zugegeben. Nach 12stündigem Rühren bei 20°C wird das Gemisch eingeengt. Der Rückstand wird mit 200 ml 20 %iger Kalilauge 1 Stunde gerührt und mit 500 ml Methylenchlorid extrahiert. Die organische Phase wird dreimal mit je 50 ml Wasser gewaschen, über Natriumsulfat getrocknet und eingeengt. Der Rückstand kristallisiert bei +5°C nach Zugabe von 20 ml Ether. Man isoliert 45 g (49,6 %) 1-(5-Methyl-1,3-dioxan-5-yl)-2-(1,2,4-triazolyl-(1))-3-(4-chlorphenyl)-propan-1-ol als weiße Kristalle vom Schmelzpunkt 152 bis 154°C.

Beispiel 3

Unter kräftigem Rühren wird eine Mischung aus 15,2 g (0,045 Mol) 1-(5-Methyl-1,3-dioxan-5-yl)-2-(1,2,4-triazolyl-(1))-3-(4-chlorphenyl)-propan-1-ol, 100 g 1-Chlorpropan, 3 g Tetrabutylammoniumhydrogensulfat und 65 g 50 %iger Natronlauge 36 Stunden auf 30°C erwärmt. Die Mischung wird sodann mit 300 ml Wasser versetzt und zweimal mit je 150 ml Methylenchlorid extrahiert. Die vereinigten Extrakte werden achtmal mit je 100 ml Wasser ausgeschüttelt, über Magnesiumsulfat getrocknet und eingeengt. Der Rückstand kristallisiert nach Zugabe von 20 ml Petrolether und 5 ml Ether. Man isoliert 12 g (70,3 %) 1-(5-Methyl-1,3-dioxan-5-yl)-1-n-propoxy-2-(1,2,4-triazolyl-(1))-3-(4-chlorphenyl)-propan als weiße Kristalle vom Schmelzpunkt 94 bis 96°C.

Beispiel 4

Zu einer Suspension von 2,4 g Natriumhydrid in 120 ml trockenem Tetrahydrofuran wird die Lösung von 23,6 g (0,07 Mol) 1-(5-Methyl-1,3-dioxan-5-yl)-2-(1,2,4-triazolyl-(1))-3-(4-chlorphenyl)-propan-1-ol in 100 ml Tetrahydrofuran getropft. Nach 12stündigem Rühren bei 25°C wird das Gemisch mit der Lösung von 9,7 g (0,08 Mol) Allylbromid in 20 ml Tetrahydrofuran zugetropft. Nach 36stündigem Rühren wird das Reaktionsgemisch vorsichtig mit 20 ml Wasser versetzt und eingeengt. Der Rückstand wird in 350 ml Methylenchlorid aufgenommen, dreimal mit je 100 ml Wasser gewaschen, die organische Phase getrocknet und eingeengt. Der Rückstand wird mit 30 ml Petrolether und 10 ml Ether über Nacht bei +3°C gelassen. Der kristalline, farblose Niederschlag wird abgesaugt, mit Petrolether gewaschen und trocknet. Man erhält 22,6 g (87,5 %) 1-(5--Methyl-1,3-dioxan-5-yl)-1-allyloxy-2-(1,2,4-triazolyl-

-(1))-3-(4-chlorphenyl)-propan vom Schemlzpunkt 113 bis 115°C.

Beispiel 5

Eine Mischung aus 20 g (0,053 Mol) 1-(5-Methyl-1,3-dioxan-5-yl)-2-(1,2,4-triazolyl-(1))-3-(2,4-dichlorphenyl)-propan-1-ol (vgl. Beispiel 35), 2 g Imidazol und 100 ml Propionanhydrid werden 10 Stunden bei 60°C gerührt und anschließend im Vakuum eingeengt. Der Rückstand wird in 250 ml Ether gelöst und 30 Minuten mit 100 ml einer 6 %igen Natriumhydrogencarbonat-Lösung gerührt. Die organische Phase wird über Natriumsulfat getrocknet und im Vakuum - schließlich bis 50°C und 0,1 mbar - eingeengt. Man erhält 17,1 g (75,4 %) 1-(5-Methyl-1,3-dioxan-5-yl)-1-acetoxy-(1,2,4-triazolyl-(I))-3-(2,4-dichlorphenyl)-propan als hellbraunes Harz.

$^1$H-NMR (80 MHz/$CDCl_3$): = 0,85 (s, 3H), 1,15-1,4 (t, 3H), 2,4-3,2 (q, 2H), 3,3-3,6 (m, 2H), 3,8-4,2 (m, 2H), 4,6-5,1 (2 dd, zus. 2H), 6,1 (s, 1H), 6,5-7,6 (2 tt, zus. 4H), 8,1 (s, 1H) und 8,28 ppm (s, 1H).

Beispiel 6

Unter Wasserabscheidung am Rückfluß wird eine Mischung aus 12,7 g (0,06 Mol) 1-(5-Methyl-1,3-dioxan-5-yl)-2-(1,2,4-triazolyl-(1))-ethan-1-on, 10,8 g (0,06 Mol) 2,4-Dichlorbenzaldehyd, 1 g Piperidin, 0,5 g Essigsäure und 150 ml Toluol 10 Stunden gerührt. Das Gemisch wird abgekühlt, dreimal mit je 80 ml Wasser gewaschen, über Natriumsulfat getrocknet und im Vakuum eingeengt. Der Rückstand wird in 250 ml Acetonitril aufgelöst, bei 40°C mit 1 g Tierkohle entfärbt, auf 40 ml eingeengt und auf 0°C abgekühlt. Der farblose Niederschlag wird abgesaugt,

mit 40 ml Ether gewaschen und getrocknet. Man erhält 18,4 g (83,3 %) 1-(5-Methyl-1,3-dioxan-5-yl)-2-(1,2,4-triazolyl-(1))-3-(2,4-dichlorphenyl)-propen-1-on vom Schmelzpunkt 133-135°C.

Beispiel 7

Zu einer Lösung von 36,1 g (0,1 Mol) 1-(5-Methyl-1,3-dioxan-5-yl)-2-(1,2,5-triazolyl-(1))-3-(2,4-dichlorphenyl)-propen-1-on in 250 ml Methanol werden zwischen -5 und 0°C 4,5 g (0,118 Mol) Natriumborhydrid portionsweise zugegeben. Nach 14stündigem Rühren bei 22°C wird der weiße Niederschlag abgesaugt und das Filtrat im Vakuum eingeengt. Niederschlag und Rückstand werden vereinigt und 0,5 Stunden mit 3 l Methylenchlorid um 500 ml 10 %iger Kalilauge bei 25°C gerührt. Die organische Schicht wird abgetrennt, dreimal mit je 100 ml Wasser gewaschen, über Natriumsulfat getrocknet und eingeengt. Der Rückstand kristallisiert nach Zugabe von 10 ml Ether. Man erhält 31,3 g (84,6 %) 1-(5-Methyl-1,3-dioxan-5-yl)-2-(1,2,4-triazolyl-(1))-3-(2,4-dichlorphenyl)-propen-1-ol als farblose Kristalle vom Schmelzpunkt 190-192°C.

Beispiel 8

Eine Mischung aus 14 g (0,0378 Mol) 1-(5-Methyl-1,3-dioxan-5-yl)-2-(1,2,4-triazolyl-(1))-3-(3,4-dichlorphenyl)-propen-1-ol (Beispiel 25) 1 g Imidazol und 80 g Acetanhydrid werden 8 Stunden bei 70°C gerührt und anschließend im Vakuum eingeengt. Der Rückstand wird in 300 ml Ether aufgelöst und 0,5 Stunden mit 100 ml einer 6 %igen Natriumhydrogencarbonat-Lösung gerührt. Die organische Phase wird über Natriumsulfat getrocknet und im Vakuum - schließlich bis 50°C und 0,1 mbar - eingeengt. Man erhält 10,8 g (69,3 % der Theorie) 1-(5-Methyl-1,3-dioxan-5-yl)-1-acet-

oxy-2-(1,2,4-triazolyl-(1))-3-(3,4-dichlorphenyl)-propen-2 als blaßgelbes Harz.

$^1$H-NMR (80 MHz/$CDCl_3$): = 0,6 (s, 3H), 2,08 (s, 3H), 3,2--3,4 (m, 2H), 3,8-4,0 (g, 2H), 4,4-4,9 (2dd, zus. 2H), 5,5 (s, 1H), 6,3-7,4 (m, zus. 4H), 8,0 (s, 1H) und 8,2 ppm (s, 1H).

In entsprechender Weise können die in der folgenden Tabelle aufgeführten Verbindungen hergestellt werden.

| Beispiel Nr. | Ar | $R^1$ | $R^2$ | X | Z + Y | Fp/°C | IR (Film) [$cm^{-1}$] |
|---|---|---|---|---|---|---|---|
| 9 | $C_6H_5-$ | $CH_3-$ | H | -CO- | $-CH_2-CH-$ | 96- 98 | |
| 10 | $C_6H_5-$ | $CH_3-$ | H | OH<br>-CH- | " | 172-174 | |
| 11 | -Naphthyl | $CH_3-$ | H | -CO- | " | 97- 99 | |
| 12 | " | $CH_3-$ | H | OH<br>-CH- | " | 174-176 | |
| 13 | " | $CH_3-$ | H | $O-COCH_3$<br>-CH- | " | 148-150 | |
| 14 | " | $CH_3-$ | $C_2H_5-$ | -CO- | " | 128-129 | |
| 15 | $C_6H_5-$ | $CH_3-$ | H | -CO- | -CH=C- | Harz | 3100,2965,1660,1585,<br>1485,1195,1150,1065,<br>920,750,690,668. |
| 16 | 1-Furanyl | $CH_3-$ | H | -CO- | " | 108-110 | |
| 17 | " | $CH_3-$ | H | OH<br>-CH- | " | 99-102 | |
| 18 | α-Naphthyl | $CH_3-$ | H | -CO- | " | 118-125 | |
| 19 | " | $CH_3-$ | $C_2H_5-$ | -CO- | " | Harz | 3112,2970,2850,1668,<br>1490,1380,1265,1155,<br>1080,918,792,770,665. |

| Beispiel Nr. | Ar | $R^1$ | $R^2$ | X | Z + Y | Fp/°C | IR (Film) [$cm^{-1}$] |
|---|---|---|---|---|---|---|---|
| 20 | α-Naphthyl | $CH_3$- | $C_2H_5$- | OH<br>-CH- | -CH=C- | Harz | 3300,3060,2965,2850, 1508,1468,1275,1160, 1090,925,880,778,675. |
| 21 | p-$C_6H_5$-$C_6H_4$ | $CH_3$- | H | -CO- | " | Harz | 3100,3010,2845,1668, 1590,1488,1150,1015, 920,910,830,755,725, 690,665. |
| 22 | " | $CH_3$- | H | OH<br>-CH- | " | 122-124 | |
| 23 | α-Naphthyl | $CH_3$- | $C_2H_5$ | OH<br>-CH- | -$CH_2$-CH- | Harz | 3105,3055,2950,2845, 1592,1365,1212,1156, 775. |
| 24 | 4F-$C_6H_4$ | $CH_3$- | H | -CO- | " | 88- 90 | |
| 25 | " | $CH_3$- | H | OH<br>-CH- | " | 140-142 | |
| 26 | 4-Cl-$C_6H_4$ | $CH_3$- | H | 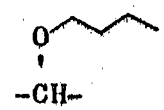 | " | 66- 68 | |
| 27 | " | $CH_3$- | $C_2H_5$- | -CO- | " | Harz | 3095,2950,2835,1695, 1472,1260,1147,1125, 1080,805,670. |

| Beispiel Nr. | Ar | $R^1$ | $R^2$ | X | Z + Y | Fp/°C | IR (Film) [$cm^{-1}$] |
|---|---|---|---|---|---|---|---|
| 28 | 4-Cl-$C_6H_4$ | $CH_3$- | $C_6H_5$- | -CO- | -CH=C- | Harz | 3022,2965,1680,1480,1376,1265,1200,1080,1000,817,750,690,666 |
| 29 | " | $CH_3$- | $C_6H_5$- | -CH(OH)- | " | Harz | 3300,2960,1478,1374,1262,1122,1080,810,750,690,666. |
| 30 | " | $CH_3$- | $C_2H_5$- | -CH(OH)- | -$CH_2$-CH- | 144-154 | |
| 31 | " | $CH_3$- | $C_2H_5$- | -CH(OH)- | " | 83- 84 | |
| 32 | " | $CH_3$- | n-$C_3H_7$- | -CO- | " | Harz | 3110,2960,2870,1712,1490,1274,1100,1022,812,705,678 |
| 33 | " | $CH_3$- | n-$C_3H_7$- | -CH(OH)- | " | 125-130 Diastereomer I | |
| 34 | " | $CH_3$- | n-$C_3H_7$- | -CH(OH)- Diastereomerengemisch | " | Harz | 3200,2960,2860,1505,1490,1268,1158,1130,1095,1020,930,825,810 |
| 35 | " | $CH_3$- | -$CH(CH_3)_2$ | CH(OH)- Diastereomer I | " | 135-155 | |

| Beispiel Nr. | Ar | $R^1$ | $R^2$ | X | Z + Y | Fp/°C | IR (Film) [$cm^{-1}$] |
|---|---|---|---|---|---|---|---|
| 36 | $4-Cl-C_6H_4-$ | $CH_3-$ | $-CH(CH_3)_2$ | $-CH(OH)-$ Diastereomerengemisch | $-CH_2-CH-$ | Harz | 3270,2960,2870,1886, 1390,12..,1130,1090, 804,950,672 |
| 37 | " | $CH_3-$ | $n-C_4H_9$ | $-CH(OH)-$ Diastereomer I | " | 123-133 | |
| 38 | " | $CH_3-$ | $n-C_4H_9$ | $-CH(OH)-$ Diastereomerengemisch | " | Harz | 2260,3095,2955,2850, 1505,140.,1274,1102, 1082,812 |
| 39 | $4-Br-C_6H_4$ | $CH_3-$ | H | -CO- | " | Harz | 3105,2965,2842,1705, 1470,13..,1253,1150, 1080,775,670 |
| 40 | " | $CH_3-$ | H | $-CH(OH)-$ | " | 156-158 | |
| 41 | $2-Cl-C_6H_4-$ | $CH_3-$ | H | -CO- | $-CH=C-$ | 86- 88 | |
| 42 | $3-Cl-C_6H_4-$ | $CH_3-$ | H | -CO- | " | Harz | 3118,3060,2980,2850, 1680,1600,1592,1270, 1155,1070,920,780, 680,665. |
| 43 | " | $CH_3-$ | H | $-CH(OH)-$ | " | Harz | 3250,3120,2845,1493, 1266,1155,1078,1023, 918,780,668. |

| Beispiel Nr. | Ar | $R^1$ | $R^2$ | X | Z + Y | Fp/°C | IR (Film) [$cm^{-1}$] |
|---|---|---|---|---|---|---|---|
| 44 | $4-Cl-C_6H_4-$ | $CH_3-$ | H | -CO- | -CH=C- | Harz | 3100,2960,2840,1665, 1572,1475,1390,1265, 1155,1082,1025,1000, 920,820,752,668. |
| 45 | " | $CH_3-$ | $C_2H_5-$ | -CO- | " | Harz | 3108,2960,2845,1685, 1578,1490,1262,1150, 1080,940,915,815,752, 665,640. |
| 46 | " | $CH_3-$ | $C_2H_5-$ | OH -CH- | " | Harz | 3380,3120,2960,2840, 1490,1480,1254,1150, 1125,1080,915,870,810, 665. |
| 47 | $4-Br-C_6H_4-$ | $CH_3-$ | H | -CO- | " | Harz | 3100,2970,2840,1665, 1567,1485,1470,1265, 1165,1060,1025,1000, 918,815,750,670. |
| 48 | " | $CH_3-$ | H | OH -CH- | " | Harz | 3400,2970,2850,1495, 1480,1270,1158,1132, 1068,1022,1004,918, 810,668. |
| 49 | " | $CH_3-$ | H | $O-COCH_3$ -CH- | -CH=C- | Harz | 3110,2960,2840,1730, 1490,1475,1360,1218, 1150,1020,915,806,662. |

| Beispiel Nr. | Ar | $R^1$ | $R^2$ | X | Z + Y | Fp/°C | IR (Film) [$cm^{-1}$] |
|---|---|---|---|---|---|---|---|
| 50 | $4-Br-C_6H_4-$ | $CH_3-$ | H | $O-COCH_2CH_3$<br>$-CH-$ | $-CH=C-$ | Harz | 3118,2970,2845,1730, 1578,1490,1478,1265, 1150,1070,1000,918, 810,665. |
| 51 | " | $CH_3-$ | $C_2H_5-$ | -CO- | " | Harz | 3115,2970,2850,1690, 1580,1492,1268,1128, 1085,1065,1002,920, 816,752,670. |
| 52 | " | $CH_3-$ | $C_2H_5-$ | OH<br>$-CH-$ | " | Harz | 3300,2960,2845,1492, 1476,1390,1265,1150, 1082,1060,1000,915, 870,810,665. |
| 53 | $3,4-DiCl-C_6H_3-$ | $CH_3-$ | H | -CO- | " | Harz | 3110,2975,2855,1680, 1600,1475,1462,1270, 1200,1155,1070,920, 880,760,665. |
| 54 | " | $CH_3-$ | H | OH<br>$-CH-$ | " | Harz | 3400,2958,2840,1490, 1460,1152,1125,1075, 1020,914,884,810,665 |
| 55 | " | $CH_3-$ | $C_2H_5-$ | -CO- | " | Harz | 3105,2970,2850,1690, 1430,1450,1380,1265, 1150,1080,1022,920, 870,810,665. |
| 56 | $4-Br-C_6H_4-$ | $CH_3-$ | $C_2H_5-$ | -CO- | $-CH_2-CH-$ | Harz | 3102,2960,2920,2845, 1705,1475,1390,1260, 1150,1080,800,668. |

| Beispiel Nr. | Ar | $R^1$ | $R^2$ | X | Z + Y | Fp/°C | IR (Film) [$cm^{-1}$] |
|---|---|---|---|---|---|---|---|
| 57 | $4-Br-C_6H_4-$ | $CH_3-$ | $C_2H_5-$ | OH<br>-CH- | $-CH_2-CH-$ | 172-173 | |
| | | | | Diastereomer I | | | |
| 58 | " | $CH_3-$ | $C_2H_5-$ | OH<br>-CH- | $-CH_2-CH-$ | Harz | 3260,3020,2960,2850,<br>1495,1385,1260,1125,<br>1080,775,670. |
| | | | | Diastereomerengemisch | | | |
| 59 | $3,4-DiCl-C_6H_3$ | $CH_3-$ | $C_2H_5-$ | -CO- | $-CH_2-CH-$ | Harz | 3106,2965,2850,1705,<br>1460,1385,1265,1125,<br>1024,918,810,750. |
| 60 | " | $CH_3-$ | $C_2H_5-$ | OH<br>-CH- | " | 171-173 | |
| | | | | Diastereomer I | | | |
| 61 | " | $CH_3-$ | $C_2H_5-$ | OH<br>-CH- | " | Harz | 3200,2985,2840,1460,<br>1384,126.,1120,1020,<br>910,815,692 |
| | | | | Diastereomerengemisch | | | |
| 62 | $2,4,5-TriCl-C_6H_2-$ | $CH_3-$ | H | -CO- | $-CH_2-CH-$ | 118-120 | |
| 63 | $2,4-DiCl-C_6H_3-$ | $CH_3-$ | H | OH<br>-CH- | " | 133-136 | |
| 64 | " | $CH_3-$ | $C_2H_5-$ | -CO- | " | Harz | 3100,2955,2840,1698,<br>1570,1458,1260,1150,<br>1125,1090,920,825 |

| Beispiel Nr. | Ar | $R^1$ | $R^2$ | X | Z + Y | Fp/°C | IR (Film) [$cm^{-1}$] |
|---|---|---|---|---|---|---|---|
| 65 | 2,4-DiCl-$C_6H_3$- | $CH_3$- | $C_2H_5$- | -CH(OH)- | -$CH_2$-CH- | 138-143 | |
| 66 | " | $CH_3$- | $C_6H_5$- | -C(=O)- | -CH=C- | Harz | 3040,2960,1678,1486, 1372,1260,1200,1080, 980,850,820,740,688. |
| 67 | " | $CH_3$- | $C_6H_5$- | -CH(OH)- | " | Harz | 3300,2950,1572,1455, 1370,1262,1120,1080, 980,960,850,810,740, 688. |
| 68 | " | $CH_3$- | n-$C_3H_7$- | -CO- | -$CH_2$-CH- | Harz | 3100,2950,2860,1705, 1580,1466,1380,1268, 1096,825,672. |
| 69 | " | $CH_3$- | n-$C_3H_7$- | -CH(OH)- | " | 127-129 | |
| 70 | " | $CH_3$- | -$CH(CH_3)_2$ | -CO- | " | Harz | 3105,2950,2860,1705, 1578,1460,1380,1260, 1155,1095,922,825, 670. |
| 71 | " | $CH_3$- | -$CH(CH_3)_2$ | -CH(OH)- Diastereomer I | " | 141-143 | |
| 72 | " | $CH_3$- | -$CH(CH_3)_2$ | -CH(OH)- Diastereomerengemisch | " | | 3130,2955,2855,1735, 1466,1360,1228,1130, 1098,940,910,860 |

| Beispiel Nr. | Ar | $R^1$ | $R^2$ | X | Z + Y | Fp/°C | IR (Film) [$cm^{-1}$] |
|---|---|---|---|---|---|---|---|
| 73 | 2,4-DiCl-$C_6H_3$- | $CH_3$- | $-CH(CH_3)_2$ | $O-COCH_3$<br>$-CH-$ | $-CH_2-CH-$ | 118-128 | |
| 74 | " | $CH_3$- | n-$C_4H_9$ | -CO- | " | Harz | 3100,2950,2855,1706, 1580,1465,1264,1150, 1095,920,824,668. |
| 75 | " | $CH_3$- | n-$C_4H_9$ | OH<br>$-CH-$ | " | 153-165 | |
| 76 | " | $CH_3$- | n-$C_4H_9$ | $O-COCH_3$<br>$-CH-$ | " | Harz | 3100,2950,2850,1740, 1467,1220,1130,1100, 1025,948,905,812,67. |
| 77 | 3,4-DiCl-$C_6H_5$- | $CH_3$- | $C_2H_5$- | OH<br>$-CH-$ | $-CH=C-$ | Harz | 3300,2970,2855,1505, 1470,1400,1275,1135, 1090,1030,925,880. |
| 78 | 4-Cl-$C_6H_4$- | $C_2H_5$- | $C_2H_5$- | -CO- | " | Harz | 3108,2960,2924,2845, 1675,1480,1392,1265, 1123,1080,916,815, 775,664. |
| 79 | 2,4-DiCl-$C_6H_3$- | $C_2H_5$- | $C_2H_5$- | -CO- | " | Harz | 3100,2964,2930,2850, 1680,1576,1640,1375, 1266,1125,1095,918, 876,860,826,812,664. |
| 80 | " | $CH_3$- | $C_2H_5$- | -CO- | " | Harz | 3100,2970,2850,1680, 1575,1460,1265,1155, 1125,1090,992,918,860, 665. |

| Beispiel Nr. | Ar | $R^1$ | $R^2$ | X | Z + Y | Fp/°C | IR (Film) [$cm^{-1}$] |
|---|---|---|---|---|---|---|---|
| 81 | 2,4-DiCl-$C_6H_3$- | $CH_3$- | $C_2H_5$- | OH<br>-CH- | -CH=C- | Harz | 3300,2970,2845,1580, 1495,1460,1265,1152, 1085,1045,918 |
| 82 | " | $CH_3$- | -$CH(CH_3)_2$- | -CO- | " | Harz | 3100,2970,2860,1690, 1580,1465,1380,1270, 1185,1130,1095,995, 920,860,830,670. |
| 83 | " | $CH_3$- | -$CH(CH_3)_2$- | OH<br>-CH- | " | Harz | 3300,2960,2845,1580, 1500,1465,1268,1130, 1095,995,918,860,815, 670. |
| 84 | " | $CH_3$- | n-$C_4H_9$ | -CO- | " | Harz | 3120,2950,2860,1710, 1685,1578,1496,1462, 1380,1268,1130,1097, 945,860,770,680. |
| 85 | " | $CH_3$- | n-$C_4H_9$ | OH<br>-CH- | " | Harz | 3200,2945,2850,1498, 1467,1365,1268,1125, 1098,1018,990,925, 855,815. |
| 86 | 2,3,4-Tri-Cl-$C_6H_2$- | $CH_3$- | H | -CO- | " | 110-140 | |
| 87 | " | $CH_3$- | H | OH<br>-CH- | " | 156-159 | |
| 88 | " | $CH_3$- | H | O-$COCH_3$<br>-CH- | " | 138-140 | |

| Beispiel Nr. | Ar | $R^1$ | $R^2$ | X | Z + Y | Fp/°C | IR (Film) [$cm^{-1}$] |
|---|---|---|---|---|---|---|---|
| 89 | $3-CF_3-C_6H_4-$ | $CH_3-$ | H | -CO- | -CH=C- | Harz | 3115,2980,2850,1680, 1493,1324,1156,1118, 1068,1025,918,795,695, 670,655. |
| 90 | $3-CF_3-C_6H_4-$ | $CH_3-$ | H | OH<br>-CH- | " | 95- 98 | |
| 91 | " | $CH_3-$ | H | $O-CO-CH_2CH_3$<br>-CH- | " | Harz | 3110,2970,2840,1730, 1490,1320,1190,1150, 1115,1065,915,795, 690,665,650. |
| 92 | $4-CF_3-C_6H_4-$ | $CH_3-$ | H | -CO- | " | Harz | 3110,2980,2845,1680, 1605,1490,1315,1155, 1115,1056,915,828, 665. |
| 93 | " | $CH_3-$ | H | OH<br>-CH- | " | 108-118 | |
| 94 | $4-CH_3-C_6H_4-$ | $CH_3-$ | H | -CO- | " | 82- 84 | |
| 95 | " | $CH_3-$ | H | OH<br>-CH- | " | Harz | 3260,3120,2845,1505, 1492,1290,1150,1130, 1068,1024,930,918, 880,808,668. |
| 96 | " | $CH_3-$ | $C_2H_5-$ | -CO- | " | Harz | 3110,2965,2845,1675, 1595,1490,1380,1262, 1142,1125,1080,918, 805,666. |

| Beispiel Nr. | Ar | $R^1$ | $R^2$ | X | Z + Y | Fp/°C | IR (Film) [$cm^{-1}$] |
|---|---|---|---|---|---|---|---|
| 97 | $4-CF_3-C_6H_4-$ | $CH_3-$ | $C_2H_5-$ | -CO- | -CH=C- | Harz | 3110,2965,1680,1488, 1312,1262,1155,1115, 915,825,660. |
| 98 | " | $CH_3-$ | $C_2H_5-$ | OH<br>-CH-<br>Isomer I | " | 180-182 | |
| 99 | " | $CH_3-$ | $C_2H_5-$ | OH<br>-CH-<br>Isomerengemisch | " | | 3230,2980,1622,1507, 1320,1170,1130,1066, 1020,925,883,825,672 |
| 100 | $2,4-DiCl-C_6H_3-$ | $CH_3-$ | $-C(CH_3)_3$ | -CO- | $-CH_2-CH-$ | 165-168 | |
| 101 | " | $CH_3$ | $-C(CH_3)_3$ | OH<br>-CH- | " | Harz | 3320,2960,2860,1465, 1267,1188,1128,1095, 860,820,692. |
| 102 | $3-CH_3-C_6H_4-$ | $CH_3-$ | H | -CO- | " | 53- 55 | |
| 103 | " | $CH_3-$ | H | OH<br>-CH- | " | 128-130 | |
| 104 | $4-CH_3-C_6H_4-$ | $CH_3-$ | H | -CO- | " | 112-114 | |
| 105 | " | $CH_3-$ | H | OH<br>-CH- | " | 114-116 | |
| 106 | " | $CH_3-$ | $C_2H_5$ | -CO- | " | Harz | 3110,2965,2850,1706, 1490,1380,1265,1155, 1082,915,800,630. |
| 107 | " | $CH_3-$ | $C_2H_5-$ | OH<br>-CH-<br>Diastereomer I | " | 161-162 | |

| Beispiel Nr. | Ar | $R^1$ | $R^2$ | X | Z + Y | Fp/°C | IR (Film) [$cm^{-1}$] |
|---|---|---|---|---|---|---|---|
| 108 | $4-CH_3-C_6H_4-$ | $CH_3-$ | $C_2H_5-$ | -CH(OH)- | $-CH_2-CH-$ | Harz | 3280,2965,2845,1500, 1385,1262,1190,1080, 918,870,800,670. |
| | | | | Diastereomerengemisch | | | |
| 109 | $4-C_2H_5-C_6H_4-$ | $CH_3-$ | H | -CO- | " | 59- 60 | |
| 110 | " | $CH_3-$ | H | -CH(OH)- | " | 111-114 | |
| 111 | $4-CH_3-C_6H_4-$ | $CH_3$ | $C_2H_5-$ | -CH(OH)- | -CH=C- | Harz | 3300,2960,2840,1500, 1455,1390,1265,1154, 1128,1084,1022,918, 872,805,668. |
| 112 | $4-t-butyl-C_6H_4-$ | $CH_3-$ | H | -CO- | " | Harz | 3118,2960,2860,1670, 1590,1490,1355,1260, 1165,1065,1025,995, 915,825,750,668. |
| 113 | " | $CH_3-$ | H | -CH(OH)- | " | Harz | 3300,2955,2850,1492, 1450,1352,1260,1155, 1072,1025,918,820,668 |
| 114 | $4-CH_3O-C_6H_4-$ | $CH_3-$ | H | -CO- | " | 132-134 | |
| 115 | " | $CH_3-$ | $C_2H_5-$ | -CO- | " | Harz | 3115,2970,2840,1670, 1590,1505,1250,1170, 1165,1125,1080,1020, 918,826,668. |
| 116 | " | $CH_3-$ | $C_2H_5-$ | -CH(OH)- | " | Harz | 3320,2840,1595,1500, 1455,1385,1240,1080, 1020,915,870,820,668 |
| 117 | $4-C_2H_5O-C_6H_4-$ | $CH_3-$ | H | -CO- | " | 117-120 | |

| Beispiel Nr. | Ar | $R^1$ | $R^2$ | X | Z + Y | Fp/°C | IR (Film) [$cm^{-1}$] |
|---|---|---|---|---|---|---|---|
| 118 | $4-C_2H_5O-C_6H_4-$ | $CH_3-$ | H | OH<br>-CH- | -CH=C- | 123-125 | |
| 119 | " | $CH_3-$ | $C_2H_5-$ | -CO- | " | Harz | 3115,2980,1690,1590,<br>1500,1383,1250,1174,<br>1130,1084,1035,920,<br>830,672. |
| 120 | " | $CH_3-$ | $C_2H_5-$ | OH<br>-CH- | " | Harz | 3300,2970,1600,1500,<br>1240,1182,1085,1038,<br>918,875,816,668. |
| 121 | $4-NO_2-C_6H_4-$ | $CH_3-$ | H | -CO- | " | Harz | 3110,2975,2850,1680,<br>1590,1510,1340,1155,<br>1025,915,840,742,668 |
| 122 | " | $CH_3-$ | H | OH<br>-CH-<br>Isomer I | " | 201-203 | |
| 123 | " | $CH_3-$ | H | OH<br>-CH-<br>Isomeren-<br>gemisch | " | Harz | 3250,2850,1590,1510,<br>1335,1152,1130,1070,<br>1020,918,842,749,690,<br>665. |
| 124 | " | $CH_3$ | $C_2H_5-$ | -CO- | " | Harz | 3115,2975,1690,1595,<br>1515,1342,1225,1160,<br>1130,1000,922,852,<br>820,745- |
| 125 | " | $CH_3-$ | $C_2H_5-$ | OH<br>-CH- | " | Harz | 3300,2960,1590,1510,<br>1336,1270,1130,1083,<br>918,873,845,735. |

| Beispiel Nr. | Ar | $R^1$ | $R^2$ | X | Z + Y | Fp/°C | IR (Film) [$cm^{-1}$] |
|---|---|---|---|---|---|---|---|
| 126 | $C_6H_5$ | $CH_3-$ | $C_2H_5-$ | -CO- | -CH=C- | Harz | 3120,2975,1678,1495, 1445,1382,1268,1158, 1130,1086,922,755, 695,672. |
| 127 | " | $CH_3-$ | $C_2H_5-$ | OH -CH- | " | Harz | 3280,2960,1492,1450, 1268,1155,1130,1085, 920,874,740,692,668. |
| 128 | 3,4-Di$CH_3$-$C_6H_3$- | $CH_3-$ | H | -CO- | $-CH_2-CH-$ | Harz | 3118,2970,2855,1710, 1492,1267,1155,1078, 920,807,672. |
| 129 | " | $CH_3-$ | H | OH -CH- | " | 127-128 | |
| 130 | 4-Cl-$C_6H_4$- | $CH_3-$ | $-CH(CH_3)_2$ | -CO- | " | Harz | 3100,2960,2840,1710, 1270,1120,1080,1025, 810,705,678. |
| 131 | " | $CH_3-$ | n-$C_4H_9$- | -CO- | " | Harz | 3105,2950,2845,1705, 1260,1150,1125,1080, 1022,808,670. |
| 132 | " | $C_2H_5-$ | $C_2H_5-$ | -CO- | " | 89- 91 | |
| 133 | " | $C_2H_5-$ | $C_2H_5-$ | OH -CH- | " | 115-117 | |
| 134 | 2,4-DiCl-$C_6H_3$- | $C_2H_5-$ | $C_2H_5-$ | -CO- | " | 61- 63 | |
| 135 | 4-Cl-$C_6H_4$- | $C_2H_5-$ | $C_2H_5-$ | OH -CH- | " | 113-115 | |

| Beispiel Nr. | Ar | $R^1$ | $R^2$ | X | Z + Y | Fp/°C | IR (Film) [$cm^{-1}$] |
|---|---|---|---|---|---|---|---|
| 136 | $4-F_3C-C_6H_4$ | $CH_3$ | H | -CO- | -CH=C- | 97-99 | |
| 137 | " | $CH_3$ | H | -CH(OH)- | " | 161-164 | |

Die erfindungsgemäßen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz, u.a. zur Bekämpfung holzzerstörender Pilze, wie Coniophora puteana und Polystictus versicolor, verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse – wie Gurken, Bohnen und Kürbisgewächse –.

Die neuen Verbindungen sind insbesondere geeignet zur Bekämpfung folgender Pflanzenkrankheiten:

Erysiphe graminis (echter Mehltau) in Getreide,
Erysiphe cichoriacearum an Kürbisgewächsen,
Podosphaera leucotricha an Äpfeln,
Uncinula necator an Reben,
Puccinia-Arten an Getreide,
Rhizoctonia solani an Baumwolle
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Phtophthora infestans (falscher Mehltau) an Kartoffeln, Tomaten.

Die Verbindungen werden angewendet, indem man die Pflanzen mit den Wirkstoffen besprüht oder bestäubt oder die Samen

der Pflanzen mit den Wirkstoffen behandelt. Die Anwendung erfolgt vor oder nach der Infektion der Pflanzen oder Samen durch die Pilze.

Die erfindungsgemäßen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen - Fettalkohol - Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha.

Die neuen Verbindungen können auch im Materialschutz u.a. zur Bekämpfung holzzerstörender Pilze, wie Coniophora puteana und Polystictus versicolor eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5 Gew.% Wirkstoff, bezogen auf das Gesamtgewicht der zu konservierenden Farben bzw. des mikrozidauszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

I. 20 Gewichtsteile der Verbindung des Beispiels 7 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

II. 3 Gewichtsteile der Verbindung des Beispiels 28 werden mit 97 Gewichtsteilen feinteiligem Kaolin innig vermischt. Man erhält auf diese Weise ein Stäubemittel, das 3 Gew.% des Wirkstoffs enthält.

III 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 40 Gewichtsteile der Verbindung des Beispiels 34 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

V 20 Teile der Verbindung des Beispiels 46 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI Man vermischt 90 Gewichtsteile der Verbindung des Beispiels 1 mit 10 Gewichtsteilen N-Methyl-$\alpha$-pyrrolidon und erhält eine Lösung, die zur Anwendung in Form kleinster Tropfen geeignet ist.

VII 20 Gewichtsteile der Verbindung des Beispiels 29 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calcium-

salz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

VIII 20 Gewichtsteile der Verbindung des Beispiels 38 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IX 10 Gewichtsteile der Verbindung des Beispiels 101, 20 Gewichtsteile Polyoxyethylensorbitan-monolaurat (®Tween 20), 20 Gewichtsteile Methanol und 50 Gew.-Teile Wasser werden zu einer Lösung verrührt, die 10 Gew.% des Wirkstoffs enthält. Durch Hinzufügen von weiterem Wasser können stärker verdünnte Lösungen hergestellt werden.

Die erfindungsgemäßen Mittel können in diesen Aufwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des Wirkungsspektrums. Bei einer Anzahl solcher Fungizidmischungen treten auch synergistische Effekte auf, d.h. die Wirksamkeit des Kombinationsproduktes ist größer als die ad-

dierten Wirksamkeiten der Einzelkomponenten.

Fungizide, die mit den erfindungsgemäßen Verbindungen kombiniert werden können, sind beispielsweise Dithiocarbamate und deren Derivate, wie
Ferridimethyldithiocarbamat,
Zinkdimethyldithiocarbamat,
Manganethylenbisthiocarbamat,
Mangan-Zink-ethylendiamin-bis-dithiocarbamat,
Zinkethylenbisthiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zinn-(N,N'-propylen-bis-dithiocarbamat) und
N,N'-Polypropylen-bis-(thiocarbamoyl)-disulfid;

Nitrophenolderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazol-acetat,
2,4-Dichlor-6-(o-chloranilino)-s-triazin,
0,0-Diethyl-phthalimidophosphonthionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithiaanthrachinon,
2-Thio-1,3-dithio-(4,5-b)-chinoxalin,

1-Butylcarbamoyl-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thiol-1-oxid,
8-Hydrochinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-2))-benzimidazol,
Piperazin-1,4-diyl-bis-1-(2,2,2-trichlor-ethyl)-formamid,
2-Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie
Dodecylguanidinacetat,
3-(2-(3,5-Dimethyl-2-oxycyclohexyl)-2-hydroxyethyl)-glutarimid,
Hexachlorbenzol,
N-Dichlorfluormethylthio-N,N'-dimethyl-N-phenyl-schwefelsäurediamid,
D,L-Methyl-N-(2,6-dimethyl-phenyl)-N-furyl(2)-alaninat,
D,L-N-(2,6-Dimethyl-phenyl)-N-(2'-methoxyacetyl)-alaninmethylester,
5-Nitro-isophthalsäure-di-isopropylester,
2,5-Dimethyl-furan-3-carbonsäureanilid,
2,5-Dimethyl-furan-3-carbonsäure-cyclohexylamid,
2-Methyl-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,

1,4-Dichlor-2,5-dimethoxybenzol, p-Dimethylaminobenzol-diazinnatriumsulfonat, 1-Chlor-2-nitro-propan,

Polychlornitrobenzole, wie Pentachlornitrobenzol, Methylisocyanat, fungizide Antibiotika, wie Griseofulvin oder Kasugamycin, Tetrafluordichloraceton, 1-Phenylthiosemicarbazid, Bordeauxmischung, nickelhaltige Verbindungen und Schwefel.

Die folgenden Beispiele zeigen die biologische Wirkung der neuen Substanzen. Als Vergleichssubstanz diente 2,2-Dimethyl-4-(1,2,4-triazol-1-yl)-5-phenylpentan-3-on (X) (DE-OS 26 38 470).

Beispiel A

Blätter von in Töpfen gewachsenen Weizenkeimlingen der Sorte "Jubilar" werden mit wäßrigen Emulsionen aus 80 % (Gew.%) Wirkstoff und 20 % Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltauentwicklung ermittelt. In diesem Versuch zeigten insbesondere die Substanzen der Beispiele 2, 3, 5, 10, 25, 30, 33, 34, 40, 63, 65, 75 und 105 eine bessere Wirkung als X.

Beispiel B

Blätter von in Töpfen gewachsenen Weizensämlingen der _orte "Caribo" werden mit Sporen des Braunrostes (Puccinia recondita) bestäubt. Danach werden die Töpfe für 24 Std.

bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit einer 0,025 %igen (Gew.%) wäßrigen Spritzbrühe, die 80 % Wirkstoff und 20 % Ligninsulfonat in der Trockensubstanz enthält, tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittelt.

In diesem Versuch zeigten insbesondere die neuen Substanzen 29 und 63 eine bessere Wirkung als X.

Beispiel C

Blätter von in Töpfen gewachsenen Gurkenkeimlingen werden mit wäßrigen Emulsionen aus 80 % Wirkstoff und 20 % Emulgiermittel besprüht und nach dem Antrocknen des Spritzbelages mit Oidien (Sporen) des Gurkenmehltaus (Erysiphe cichoracearum) bestäubt. Die Versuchspflanzen wurden anschließend im Gewächshaus bei Temperaturen zwischen 20 und 22°C und 75 bis 80 % relativer Luftfeuchtigkeit aufgestellt. Nach 10 Tagen wird das Ausmaß der Mehltaupilzentwicklung ermittelt.

In diesem Versuch zeigten insbesondere die Substanzen der Beispiele 2, 3, 5, 7, 25, 28-38, 43, 46, 52, 54, 57, 58, 60, 62-69, 71, 72, 75, 79, 81, 87, 90, 91, 93, 105, 107, 108, 110, 120, 122, 125, 127, 132-135 eine bessere Wirkung als X.

Beispiel D

Blätter von in Töpfen gewachsenen Weizenpflanzen der Sorte "Jubilar" werden mit wäßrigen Spritzbrühen, die 0,1 % (Gew.--Prozent) Wirkstoff emulgiert enthalten, tropfnaß gespritzt. Nach dem Antrocknen des Spritzbelages werden die Pflanzen mit einer Sporenaufschwemmung des Pilzes Septoria nodorum besprüht und bei 16 bis 18°C in eine Kammer mit hoher Luftfeuchtigkeit gestellt, um optimale Bedingungen für die Pilzentwicklung zu erhalten. Nach 14 Tagen hat sich die Krankheit auf den unbehandelten Kontrollpflanzen so stark entwickelt, daß die entstandenen Blattflecken den üerwiegenden Teil der Blätter bedecken.

In diesem Versuch zeigten insbesondere die Substanzen der Beispiele 73 und 86 bessere Eigenschaften als X.

Beispiel E

Paprikasämlinge der Sorte "Neusiedler Ideal Elite" werden, nachdem sich 4 bis 5 Blätter gut entwickelt haben, mit 0,1 % (Gew.-%) wäßrigen Suspensionen, die 80 % Wirkstoff und 20 % Natriumligninsulfonat in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt, um optimale Bedingungen für die Pilzentwicklung zu erhalten. Nach 5 Tagen hat sich die Krankheit auf den unbehandelten Kontrollpflanzen so stark entwickelt, daß die entstandenen Blattnekrosen den überwiegenden Teil der Blätter bedecken.

In diesem Versuch zeigten insbesondere die Substanzen der Beispiele 34, 40, 47 und 63 bessere Wirkungen als X.

Beispiel F

Blätter von Tomatenpflanzen der Sorte "Professor Rudloff" werden mit wäßrigen Suspensionen, die 80 % (Gew.%) des zu prüfenden Wirkstoffs und 20 % Natriumligninsulfonat in der Trockensubstanz enthalten, besprüht. Es werden 0,05 und 0,025 %ige Spritzbrühen (berechnet auf die Trockensubstanz) verwendet. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

In diesem Versuch zeigten insbesondere die Substanzen der Beispiele 40 und 63 eine bessere Wirkung als X.

Patentansprüche

1. Fungizide Mittel, enthaltend ein Triazolylderivat der Formel

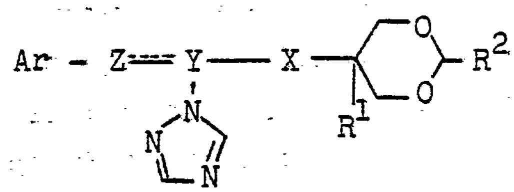

I

in der

$R^1$ und $R^2$ Wasserstoff, Alkyl mit 1 bis 5 C-Atomen oder Phenyl bedeuten, wobei der Phenylrest durch Fluor, Chlor, Brom, Nitro, Trifluormethyl, weiterhin durch Alkyl, Alkoxy oder Alkenyl mit jeweils 1 bis 3 C-Atomen substituiert sein kann und

Ar Furanyl, Thienyl, Biphenylyl, Naphthyl oder Phenyl bedeutet, wobei der Phenylrest durch Fluor, Chlor, Brom, Iod, Nitro, Trifluormethyl, weiterhin durch Alkyl, Alkoxy oder Alkenyl mit jeweils 1 bis 5 C-Atomen und ferner durch Phenoxy substituiert sein kann,

Z⩵Y die Bedeutung CH=C oder $CH_2$-CH haben und

X eine -CO-, -CH(OH)- oder -CH($OR^3$)-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet - wobei der Benzylrest durch Fluor, Chlor, Brom, Nitro, Trifluormethyl; ferner durch Alkyl oder Alkoxy

mit 1 bis 3 C-Atomen substituiert sein kann - oder $R^3$ einen $-CO-R^4$-Rest bedeutet, wobei $R^4$ einen gegebenenfalls durch Halogen, Alkoxy, Oxo (=O) oder Carboxyalkyl substituierten Alkylrest mit 1 bis 5 C-Atomen oder einen aromatischen Rest bedeutet, sowie deren Salze und Metallkomplexe.

2. Fungizide Mittel, enthaltend als Triazolylderivate der Formel I solche, in denen

$R^1$ und $R^2$ Wasserstoff, Methyl, Ethyl, n-Propyl, iso-Propyl, n-Butyl, sek.-Butyl, iso-Butyl, tert.-Butyl, n-Pentyl oder einen durch Fluor, Chlor, Brom, Methyl, Ethyl, Trifluormethyl, Methoxy, Ethoxy und Nitro substituierten Phenylrest bedeuten,

Ar 2-Furanyl, 2- und 3-Thienyl, 4-Biphenylyl, 1- und 2-Naphthyl, Phenyl, 4-Fluorphenyl, 2-Chlorphenyl, 3-Chlorphenyl, 4-Chlorphenyl, 4-Bromphenyl, 2,4-Dichlorphenyl, 3,4-Dichlorphenyl, 3,5-Dichlor-2-methoxyphenyl, 4-Ethoxyphenyl, 4-tert.-Butylphenyl, 4-Trifluormethylphenyl und 4-Phenoxyphenyl bedeutet,

Z---Y die Bedeutung CH=C oder $CH_2$-CH haben und

X eine -CO-, -CH(OH)- oder $-CH(OR^3)$-Gruppe bedeutet, wobei $R^3$ bevorzugt für Methyl, Ethyl, n-Propyl, n-Butyl, 4-Chlorbutyl, n-Pentyl, n-Hexyl, 2-Buten-1-yl, Propargyl, Benzyl, 4-Fluorbenzyl, 4-Chlorbenzyl, 4-Brombenzyl, 2,4-Dichlorbenzyl, 4-Trifluormethylbenzyl, Acetyl, Chloracetyl, Propionyl, Butyryl, Isobutyryl, Methoxyacetyl, Acryl und Butenoyl steht.

3. Fungizides Mittel, enthaltend inerte Zusatzstoffe und ein Triazolylderivat der Formel I gemäß Anspruch 1.

4. Verfahren zur Herstellung von Triazolylderivaten der Formel I gemäß Anspruch 1, dadurch gekennzeichnet, daß man

a) 1,2,4-Triazol mit α-Bromketonen der Formel II

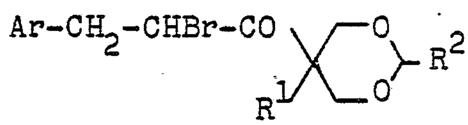
II,

worin $R^1$, $R^2$ und Ar die oben angegebene Bedeutung haben oder

b) ein Arylmethylhalogenid der Formel III

$$AR-CH_2-Y \qquad III,$$

worin Ar die oben genannte Bedeutung hat und Y Chlor oder Brom ist, mit einem 1-(1,3-Dioxan--5-yl)-2-(1,2,4-triazolyl-(s))-ethan-1-on der Formel IV

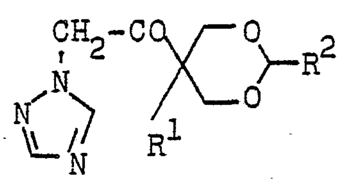
IV

worin $R^1$ und $R^2$ die oben genannte Bedeutung haben, oder

c) einen Aldehyd der Formel V

$$Ar - CH = O \qquad V,$$

in der Ar die oben genannte Bedeutung hat, mit dem 1-(1,3-Dioxan-5-yl)-2-(1,2,4-triazolyl-(1))--ethan-1-on-Derivat der Formel IV kondensiert und die so erhaltenen Verbindungen gegebenenfalls anschließend reduziert und - falls gewünscht - danach verethert oder verestert.

5. Verfahren zur Bekämpfung von Pilzen, dadurch gekennzeichnet, daß man ein Triazolylderivat der Formel I gemäß Anspruch 1 auf diese bzw. auf durch Pilzbefall bedrohte Flächen, Pfanzen oder Saatgüter einwirken läßt.

6. Verfahren zur Herstellung eines fungiziden Mittels, gemäß Anspruch 5, dadurch gekennzeichnet, daß man ein Triazolylderivat der Formel I gemäß Anspruch 1 mit festen oder flüssigen Trägerstoffen sowie gegebenenfalls mit einem oder mehreren oberflächenaktiven Mitteln mischt.